# EUROPEAN PATENT SPECIFICATION

(11) **EP 1 883 821 B1**
(45) Date of publication and mention of the grant of the patent: **04.11.2009**
(21) Application number: 05787272.3
(22) Date of filing: 24.06.2005
(51) Int. Cl.: G01N 33/68

(54) **AUTOMATED GLYCOFINGERPRINTING STRATEGY**
AUTOMATISCHE GLYKOFINGERABDRUCK-STRATEGIE
TECHNIQUE D'EMPREINTE PAR GLYCOANALYSE AUTOMATIQUE

(30) Priority: 26.04.2005 US 674724 P
(43) Date of publication of application: 06.02.2008
(73) Proprietor: National Institute for Bioprocessing Research and Training Limited, Dublin 4 (IE)
(72) Inventor: Dwek, Raymond A., University of Oxford, Oxford OX1 3QU (GB); Royle, Louise, University of Oxford, Oxford OX1 3QU (GB); Zitzmann, Nicole, University of Oxford, Oxford OX1 3QU (GB); Radcliffe, Catherine, University of Oxford, Oxford OX1 3QU (GB); Rudd, Pauline M., University of Oxford, Oxford OX1 3QU (GB)
(74) Representative: Stark, Gordon Drummond
(86) International application number: PCT/IB2005/002995
(87) International publication number: WO 2006/114663

(56) References cited:
- WO-A-2004/019040
- US-A1- 2004 253 651
- HARVEY D J: "Proteomic analysis of glycosylation: Structural determination of N- and O-linked glycans by mass spectrometry" EXPERT REVIEW OF PROTEOMICS 2005 UNITED KINGDOM, vol. 2, no. 1, 1 April 2005 (2005-04-01), pages 87-101, XP008060593 ISSN: 1478-9450
- BUTLER MICHAEL ET AL: "Detailed glycan analysis of serum glycoproteins of patients with congenital disorders of glycosylation indicates the specific defective glycan processing step and provides an insight into pathogenesis." GLYCOBIOLOGY, vol. 13, no. 9, September 2003 (2003-09), pages 601-622, XP002369864 ISSN: 0959-6658
- PERACAULA R ET AL: "Glycosylation of human pancreatic ribonuclease: Differences between normal and tumor states" GLYCOBIOLOGY, IRL PRESS,, GB, vol. 13, no. 4, 1 April 2003 (2003-04-01), pages 227-244, XP002362434 ISSN: 0959-6658

## Description

### BACKGROUND OF THE INVENTION

This invention is generally related to methods of disease diagnosis and monitoring, and, in particular, to automated methods of disease diagnostics and monitoring based on detailed glycan analysis.

Specific disease related glycosylation changes for glycans released from purified serum IgG were first reported for rheumatoid arthritis, see Parekh et. al. "Association of Rheumatoid Arthritis and Primary Osteoarthritis with Changes in the Glycosylation Pattern of Total Serum IgG, "Nature, 316, pp. 452-457, 1985. Subsequent work demonstrated that these changes were not only diagnostic of rheumatoid arthritis (RA), but could also be used as prognostic indicators as well as monitors of RA disease activity, see e.g. "Galactosylation of IgG associated oligosaccharides: Reduction in patients with adult and juvenile onset rheumatoid arthritis and relation to disease activity," R.B. Parekh, D.A. Isenberg, B.M. Ansell, I.M. Roitt, R.A. Dwek and T.W. Rademacher (1988) Lancet, 1(8592), 966-969; "A comparative analysis of disease-associated changes in the galactosylation of serum IgG" R.B. Parekh, D. Isenberg, G. Rook, I. Roitt, R.A. Dwek and T.W. Rademacher (1989) J. Autoimmunity, 2, 101-114; 3rd Jenner International Immunoglycobiology Meeting Abstract R.B. Parekh, Isenberg, D., Dwek, R.A. and Rademacher, T.W. Glycoconjugate Journal (1994) 1, 3 195-227. Later, it was demonstrated that specific glycosylation changes in total serum glycosylation can be also bio-markers of other diseases. For example, Block *et. al*. determined specific glycosylation changes in total serum of woodchucks infected with hepatitis B virus that developed hepatocellular carcinoma by performing a glycosylation analysis on glycans enzymatically released from a total serum solution, see Block, T. M., Comunale, M. A., Lowman, M., Steel, L. F., Romano, P. R., Fimmel, C., Tennant, B. C., London, W. T., Evans, A. A., Blumberg, B. S., Dwek, R. A., Mattu, T. S. and Mehta, A. S. (2005). "Use of targeted glycoproteomics to identify serum glycoproteins that correlate with liver cancer in woodchucks and humans." Proc Natl Acad Sci U S A 102: 779-84. Glycosylation analysis of whole serum glycoproteins from patients and healthy controls using a combination of high-performance liquid chromatography (HPLC) (see Guile, G. R., et. al., "A rapid high-resolution high-performance liquid chromatographic method for separating glycan mixtures and analyzing oligosaccharide profiles." Anal. Biochem. 240: 210-26, 1996; Royle, L., et. al. "An analytical and structural database provides a strategy for sequencing O-glycans from microgram quantities of glycoproteins." Anal. Biochem. 304: 70-90, 2002) and Mass Spectrometry (MS) technology was first used to confirm the diagnosis of a patient with congenital disorders of glycosylation (CDGs) type II and to establish the faulty glycosylation processing step in an undiagnosed patient, see Butler, M., et. al. "Detailed glycan analysis of serum glycoproteins of patients with congenital disorders of glycosylation indicates the specific defective glycan processing step and provides an insight into pathogenesis." Glycobiology 13: 601-22, 2003. The glycan profile and analysis were flawed because hydrazinolysis was used to release the glycans. The use of hydrazinolysis results in the desialylation of a significant proportion of the sugars and the introduction of a number of artifacts such as loss of *N*-acetyl and *N*-glycolyl groups from the amino sugar residues (which are subsequently re-*N*-acetylated and this can result in both under and over acetylation), as well as loss of O-acetyl substitutions in sialic acids. Callewaert *et. al*. used enzymatic release in whole serum glycosylation analysis by capillary electrophoresis on a microffuidic platform, see Callewaert, N., Contreras, R., Mitnik-Gankin, L., Carey, L., Matsudaira, P. and Ehrlich, D. (2004). "Total serum protein N-glycome profiling on a capillary electrophoresis-microfluidics platform." Electrophoresis 25: 3128-31 and Callewaert, N., Schollen, E., Vanhecke, A., Jaeken, J., Matthijs, G., and Contreras, R. (2003). "Increased fucosylation and reduced branching of serum glycoprotein N-glycans in all known subtypes of congenital disorder of glycosylation I." Glycobiology 13: 367-375. Although enzymatic release of Callewaert *et. al*. is compatible with a high throughput format, their analyses determined only major desialylated structures. Thus, a need still exists to develop a high throughput fully automated method for determining robust glycosylation markers of diseases based on a detailed glycosylation analysis of total glycoproteins in samples of body fluid or body tissue.

International Patent Publication No. WO 2004/019040 describes a method of identifying markers of disease states using mass spectrometry on partially purified glycan-containing samples.

US Patent Publication No. US 2004/0253651 describes a method of diagnosing cancer in a biological sample by determining the presence of cancer specific oligosaccharide sequences in the sample.

### SUMMARY OF THE INVENTION

One embodiment of the invention is an *in vitro* method of determining one or more glycosylation markers of a disease, comprising the steps of:
- providing a diseased sample and a control sample, wherein the diseased sample is a sample from a subject diagnosed with the disease and the control sample is a sample from a healthy control;
- immobilising total glycoproteins from the diseased sample and from the control sample on a protein binding membrane or in a gel without separating the gel into bands;
- releasing a glycan pool of the immobilised total glycoproteins of the diseased sample and releasing a glycan pool of the immobilised total glycoproteins of the control sample from the gel or the protein binding membrane without having purified a specific glycoprotein and without exposing the diseased sample and the control sample to hydrazinolysis;
- measuring a glycosylation profile of the glycan pool from the diseased sample and measuring a glycosylation profile of the glycan pool from the control sample using chromatography, mass spectrometry or a combination thereof; and
- comparing the glycosylation profile of the diseased sample with the glycosylation profile of the control sample to determine one or more glycosylation markers of the disease.

The sample may be a sample of a body fluid, for example, whole serum, or a body tissue. The glycans may be *N*-linked glycans or *O*-linked glycans.

The method may include a further step of labeling the glycans of the glycan pool with a radioactive or a fluorescent label prior to measuring the glycosylation profile.

The method may further comprise selecting a best glycosylation marker out of the glycosylation markers, wherein the best glycosylation marker has a highest correlation with one or more parameters of the disease.

The method may further comprise a step of segregating and/or amplifying the one or more glycosylation markers by digesting the glycans with one or more exoglycosidases. Said digesting may be performed sequentially or with an array comprising the one or more exoglycosidases.

The method may include a step of using a database to make preliminary and final assignments of structures of the glycans.

The method may further comprise a step of using the one or more glycosylation markers of the disease to diagnose, prognose and/or monitor the disease.

Another embodiment of the invention is an *in vitro* method for diagnosing and monitoring a disease in a subject comprising the steps of:
- providing a sample of body fluid or a body tissue of the subject;
- immobilising total glycoproteins from the sample on a protein binding membrane or in a gel without separating the gel into bands;
- releasing a glycan pool of the immobilised total glycoproteins of the sample from the gel or the protein binding membrane without having purified a specific glycoprotein and without exposing the sample to hydrazinolysis;
- measuring a glycosylation profile of the glycan pool; and
- determining a clinical status of the subject from one or more glycosylation markers of the disease in the glycosylation profile.

The disease may be cancer, an autoimmune disease or a congenital disorder of glycosylation. The autoimmune disease may be rheumatoid arthritis.

The body fluid may be whole serum.

In certain embodiments wherein the autoimmune disease is rheumatoid arthritis and the body fluid is whole serum, the glycosylation marker is a ratio between G0 glycans and triple-G1 glycans.

The method may further comprise a step of segregating and/or amplifying the one or more glycosylation markers by digesting the glycans with one or more exoglycosidases.

### BRIEF DESCRIPTION OF THE DRAWINGS

FIG. 1 illustrates a strategy for multidimensional glycan structural analysis.
FIG. 2 illustrates for high throughput analysis of glycans from serum glycoproteins.
FIG. 3 shows normal phase high performance liquid chromatography (NP-HPLC) profiles of 2-AB labeled *N*-glycans released from whole serum following digestion with an array of exoglycosidases.
FIG. 4 demonstrates a glycoprofile of 2-AB labeled glycans released from whole serum analysed by NP-HPLC. The glycoprofile is presented as a chromatogram using both elution times and glucose units scales.
FIG. 5 demonstrates the percentage of core fucosylated glycans in whole serum from HCV patients.
FIG. 6 shows sodium dodecyl sulphate polyacrylamide gel electrophoresis (SDS-PAGE) and NP-HPLC profiles of glycans released from purified immunoglobulin G (IgG) of samples GBRA 1 and GBRA13.
FIG. 7 shows NP-HPLC profiles of glycans released from purified IgG of sample GBRA15.
FIG. 8 shows NP-HPLC profiles of control and sample GBRA15.
FIG. 9 shows correlation between glycosylation marker of rheumatoid arthritis determined in glycans released from total serum (G0/triple G1 ratio) and rheumatoid arthritis diagnosis (determined as a percentage of G0 glycans in the total glycans released from purified IgG).
FIG. 10a-g illustrate a glycan database at Oxford Glycobiology Institute: (a) & (b) show the nomenclature used to draw the glycan structures and explain the exoglycosidase digestions; (c) shows some of the structures with GU values listed in the database; (d) to (g) follow the structure A2G2S2 (d) thorough a series of digestions, in each case the consensus GU value (which is calculated from the experimentally determined GU values listed) is given along with possible digestions and products.
FIG. 11 illustrates an algorithm for assigning glycan structures in glycoprofiles using glycan databases.

### DETAILED DESCRIPTION OF THE PREFERRED EMBODIMENTS

This invention is generally related to methods of disease diagnosis and monitoring, and, in particular, to automated methods of disease diagnosis and monitoring based on detailed glycan analysis.

Unless otherwise specified, "a" or "an" means "one or more."

"Glycoprofile" or "glycosylation profile" means a presentation of glycan structures (oligosaccharides) present in a pool of glycans. A glycoprofile can be presented, for example, as a plurality of peaks corresponding to glycan structures present in a pool of glycans.

"Glycosylation marker" means a particular difference in glycosylation between a sample of a patient diagnosed with disease and a sample from healthy control.

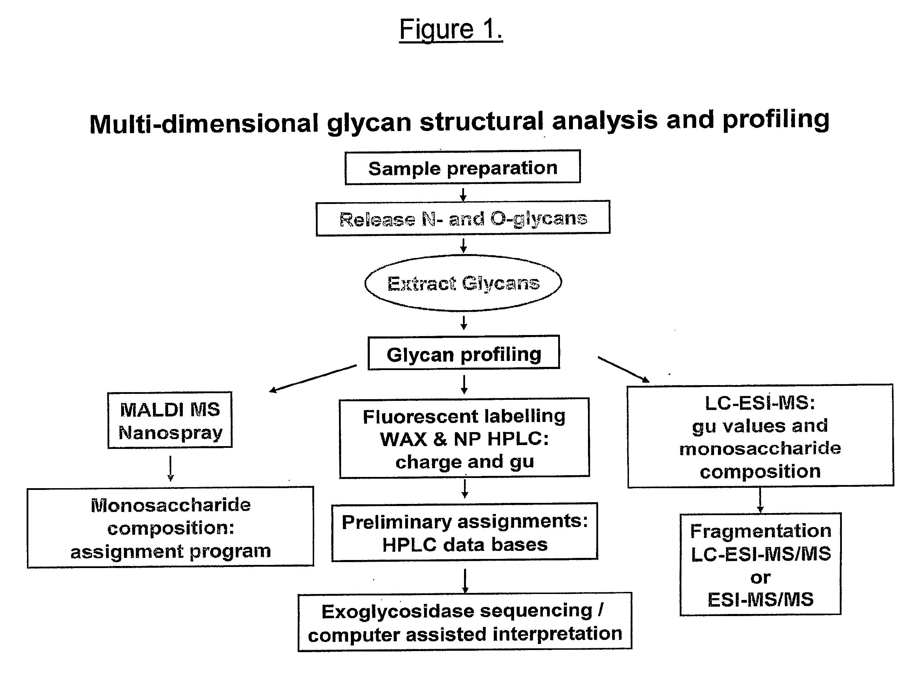

The present invention is directed to development of a fully automated system based on a detailed glycosylation analysis. The general methodology of the detailed glycosylation analysis is illustrated on Figure 1. A glycan pool (of N-linked glycans and/or O-linked glycans) of total glycoproteins, i.e. of all or substantially all glycoproteins, can be released from a sample of a body fluid or body tissue obtained from a subject. Releasing of the glycan pool is carried out without exposing the sample to hydrazinolysis and without having purified a specific glycoprotein. The detailed analysis of the glycan pool can be carried out by high performance liquid chromatography, mass spectrometry or a combination thereof. The released glycans can be fluorescently labeled prior to analysis. The detailed analysis can include separating the glycan pool into several aliquots based on the charge of glycans in each aliquot by weak anion exchange chromatography or a related technique. The NP-HPLC analysis can be performed on each aliquot. The NP-HPLC analysis performed on the total glycan pool or WAX aliquot of the glycan pool can result in a glycoprofile which can comprise a plurality of peaks. Each peak in the glycoprofile can be preliminary assigned to a particular glycan structure using a database of known glycan structures. The database can recommend particular exoglycosidase treatment to establish a final assignment of each peak in the glycoprofile.

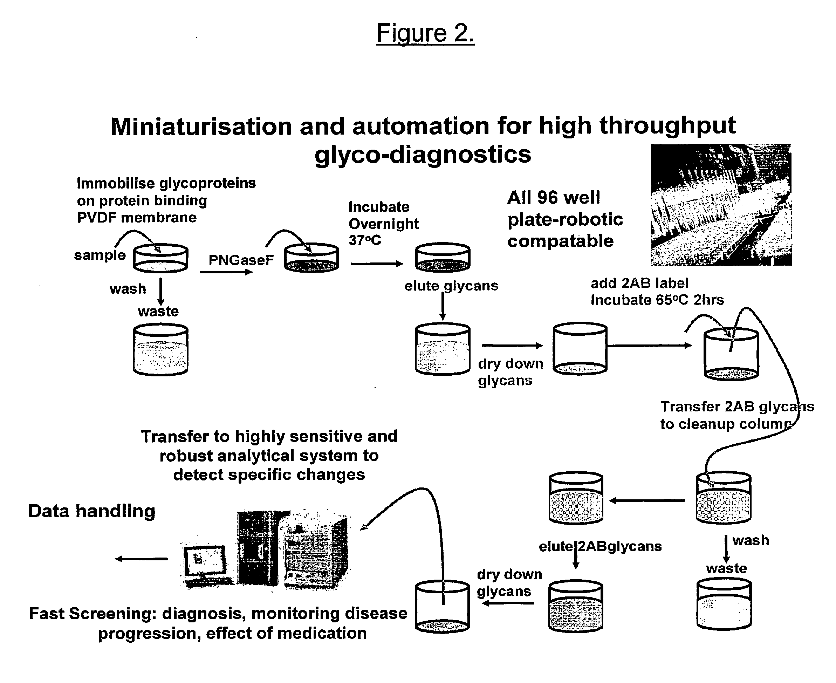

Figure 2 illustrates one possible embodiment of a high throughput fully automated system based on the detailed quantitative glycan analysis. The fully automated high throughput system should, for example, use glycan release methods fully compatible with a high throughput format and should include computer assisted data analysis suitable for clinical screening. Accordingly, the present invention provides methods of glycan releasing compatible with a high throughput format, databases and methods of using databases in automated glycan analysis. The present invention also provides methods of determining glycosylation markers of disease based on the detailed glycosylation analysis and related methods for using the glycosylation markers of disease for diagnosing and monitoring the disease.

One embodiment of the invention is an in vitro method of determining one or more glycosylation markers of disease comprising obtaining a diseased sample and a control sample, wherein the diseased sample is a sample from a patient diagnosed with disease and the control sample is a sample from a healthy control; immobilising total glycoproteins from the diseased sample and from the control sample on a protein binding membrane or in a gel without separating the gel into bands; releasing a diseased glycan pool of total immobilised glycoproteins from the diseased sample and a control glycan pool of total immobilised glycoproteins from the control sample from the gel or the protein binding membrane without having purified a specific glycoprotein and without exposing the diseased sample and the control sample to hydrazinolysis; measuring a diseased glycoprofile of the diseased glycan pool and a control glycoprofile of the control glycan pool using chromatography, mass spectrometry or a combination thereof; comparing the diseased glycoprofile and the control glycoprofiles to determine said one or more glycosylation markers of disease. In some embodiments, comparing the diseased glycoprofile and the control glycoprofile can comprise comparing peak ratios in the diseased glycoprofile and in the control glycoprofile. In some embodiments, the method of determining one or more glycosylation markers of cancer can further comprise selecting a best glycosylation marker of disease out of the one or more glycosylation marker of disease, wherein the best glycosylation marker has the highest correlation with one or parameters of the patient diagnosed with cancer. The parameters of the patient diagnosed with the disease can be, for example, diagnosis, age, sex, cancer stage, response to therapy, medical history or any combination thereof. In some embodiments, comparing the diseased glycoprofile and the control glycoprofile can be carried out following digestion of the diseased glycan pool and the control glycan pool with one or more exoglycosidase enzymes in any combination. For example, digestion of the diseased glycan pool and the control glycan pool can be a sequential digestion, digestion of the diseased glycan pool and the control glycan pool can be a digestion with an array comprising one or more exoglycosidase enzymes. In some embodiments, digestion of the diseased glycan pool and the control glycan pool with one or more glycosidase in any combination can be used to amplify and/or segregate the glycosylation markers of disease. The determined glycosylation marker can be used for diagnosing, monitoring and/or prognosticating the disease in a subject based a detailed glycosylation analysis using HPLC, mass spectrometry or a combination thereof. The determined glycosylation marker can also be used for isolating in the body fluid or the body tissue one or more glycoproteins with altered glycosylation, i.e. for isolating one or more glycoproteins that are specific biomarkers of the disease. The determined glycosylation marker can be also used for diagnosing, monitoring and/or prognosticating a disease using analytical techniques other than the techniques used to determine the glycosylation marker. These other analytical techniques can be, for example, capillary electrophoresis or lectin chromatography.

Another embodiment of the invention is an in vitro method for diagnosing and monitoring a disease in a subject comprising obtaining a sample of body fluid or a body tissue of the subject immobilising total glycoproteins from the sample on a protein binding membrane or in a gel without separating the gel into bands; releasing a glycan pool of the immobilised total glycoproteins from the sample from the gel or the protein binding membrane without having purified a specific glycoprotein and without exposing the sample to hydrazinolysis; measuring a glycoprofile of the glycan pool; and determining a clinical status of the subject from a level of a glycosylation marker of disease in the glycoprofile. In some embodiments, the glycosylation marker can be, for example, a glycosylation marker determined by the method for determining one or more glycosylation markers for disease. Measuring the glycoprofile can be carried out by any suitable technique, i.e. not necessarily by the technique used to determine the one or more glycosylation markers of the disease. For example, measuring the glycoprofile can be carried out by capillary electrophoresis or lectin chromatography.

The methods of the present invention can be directed to any disease associated with glycosylation changes such as autoimmune diseases, congenital disorders of glycosylation or cancers. The autoimmune disease can be, for example, rheumatoid arthritis, osteoarthritis, juvenile chronic arthritis, systematic lupus erythematosus, Sjögren's syndrome, ankylosing spondylitis, psoriatic arthritis, multiple sclerosis, inflammatory bowel disease, graft-vs-host disease or scleroderma. The cancer can be, for example, prostate cancer, pancreatic cancer, breast cancer, bladder cancer, renal cancer, colon cancer, ovary cancer, hepatocellular carcinoma, stomach cancer, or lung cancer. The methodology of the present invention as directed to rheumatoid arthritis and other autoimmune diseases is described in the US provisional patent application "High throughput glycan analysis for diagnosing and monitoring rheumatoid arthritis and other autoimmune diseases" to Dwek *et. al*.. The methodology of the present invention as directed to cancer is described in the US provisional application to Dwek *et. al.* "Glycosylation markers for cancer diagnostics and monitoring."

The "subject" of the foregoing embodiments is an animal, more preferably a mammal, and most preferably a human.

### Releasing glycans.

Glycans can be released from a sample of a body fluid or a body tissue, such as a sample of whole serum, blood plasma, urine, seminal fluid, seminal plasma, feces or saliva. The released glycans can be N-glycans or O-glycans. Releasing a glycan pool of glycoproteins from a sample of a body fluid or a body tissue is carried out without having purified the glycoproteins. In other words, the released glycans are glycans of all or substantially all of the glycoproteins present in the sample of a body fluid or a body tissue rather than of one or more purified and isolated glycoproteins. In some embodiments, substantially all of the glycoproteins can mean all the glycoproteins that are recovered, yet in some embodiments substantially all of the glycoproteins can mean all the glycoproteins except those that are specifically removed. Releasing glycans can be carried out without exposing a sample of a body fluid or a body tissue to hydrazinolysis. In some embodiments, releasing glycans can be carried out from a very small sample of a body fluid. In some embodiments, samples of a body fluid can be less than 100 microliters, yet preferably less than 50 microliters, yet more preferably less than 20 microliters, yet more preferably less than 10 microliters, yet most preferably less than 5 microliters. The present methods of releasing can be optimized to work with body fluid samples of less than 1 microliters.

Releasing glycans comprises immobilizing total glycoproteins of a body fluid or a body tissue, on protein binding membrane or in a gel without separating the gel into bands. The protein binding membrane can be any protein binding membrane, for example, polyvinyldene fluoride (PVDF) membrane, nylon membrane or Polytetrafluoroethylene (PTFE) membrane. Releasing glycans further comprises releasing glycans from the total glycoproteins immobilized on the protein binding membrane or in the gel. When released glycans are *N*-linked glycans, releasing glycans from the immobilized glycoproteins can be carried out using enzymatic release with, for example, peptide N glycosidase F. Releasing glycans from the gel is carried out from the total gel, i.e. without separating gel into the bands. In some embodiments, releasing glycans is carried out by chemical release methods, such as β-elimination or ammonia-based β-elhnination, which can be used for releasing *N*-linked or *O*-linked glycans from glycoproteins immobilized on protein binding membrane. For using the methods of this invention in a high throughput format, a glycan pool is released from total glycoproteins immobilized in a gel or on a protein binding membrane as it can allow the use of smaller samples of body fluid or body tissue.

The details of some of the release methods and their applicability to both N-glycans and O-glycans are discussed below, however, it should be understood that the present invention is not limited to the discussed below release methods.

*In-gel-block:* To avoid the problems with clean up of samples following solution phase enzymatic glycan release an in-gel-block release from protein mixtures can be used. Briefly, the whole protein mixture (e.g. serum or plasma) can be reduced and alkylated by adding 4µl of 5X sample buffer (5X sample buffer: 0.625ml 0.5M Tris (6g for 100ml) adjusted to pH 6.6 with HCl, 1ml 10%SDS, 0.5ml glycerol, in 2.875ml water), 2µl of 0.5M dithiothreitol (DTT) and water to make up to 20µl in total, incubated at 70°C for 10min, then alkylated by addition of 2µl of 100mM iodoacetamide and incubated for 30 min in the dark at room temperature then set into 15% SDS-gel mixture. A total volume of gel of 185 µl can be used (initially set into a 48 well plate, then removed for cutting up) with 300 µl of 100 U/ml of PNGaseF.

This gel can be washed alternatively with 1ml of acetonitrile then 1ml of digestion buffer (20mM NaHCO₃ pH 7), which can be repeated twice before the gel plug can be then dried.

The PNGaseF and gel pieces can be incubated overnight at 37°C. The supernatant can be recovered along with 3 x 200 µl water washes (with sonication with gel pieces for 30 mins each) followed by an acetonitrile wash (to squeeze out the gel), another water wash and a final acetonitrile wash. Samples can be desalted using, for example, 50 µl of activated AG-50(H⁺), filtered through a 0.45 µm LH Millipore filter and dried down for fluorescent labeling.

This procedure can be more suitable for automated glycan release than in-solution PNGaseF release, and can be the preferred method for high throughput glycan analysis. This system can be easily further modified to work with smaller amounts of gel set into a 96 well plate.

### Enzymatic release of N-glycans from PVDF membranes

The glycoproteins in reduced and denatured serum samples can be attached to a hydrophobic PVDF membrane in a 96 well plate by simple filtration. The samples can be then washed to remove contaminates, incubated with PNGaseF to release the glycans based on the methods described in Papac, D. I., et. al. Glycobiology 8: 445-54, 1998, and in Callewaert, N., et. al. Electrophoresis 25: 3128-31, 2004.

The *N*-glycans can be then washed from the bound protein, collected and dried down ready for fluorescent labeling. N-glycans can be released *in situ* from the glycoproteins by incubation with PNGaseF and by chemical means.

### Chemical release of N- and O-glycans

In contrast to the advantages that enzymatic release of N-glycans affords to N-glycan analysis, no enzymatic methodology currently exists for the release of structurally intact O-glycans. Chemical release by reductive β-elimination can require the concomitant reduction of the released oligosaccharides to their alditol derivatives (Amano, J. et. al. Methods Enzymol 179: 261-70, 1989) to prevent degradation (peeling). This reduction precludes the use of any post-release labeling so that detection is limited to mass spectrometry, pulsed amperometric detection and/or radioactivity.

*Ammonia-based* β*-elimination* can be used to release both N- and O-glycans by a modification of the classical β-elimination (Huang, Y. et. al. Analytical Chemistry 73: 6063-6069, 2001) which can be applied to glycoproteins on PVDF membranes. Ammonia-based β-elimination can be done from PVDF membranes. This strategy, can be optimized for high throughput, and can provide a powerful approach for releasing both N- and O-glycans in their correct molar proportions and in an open ring form suitable for post-release labeling.

### Release of N- and O-glycans from protein binding PVDF membranes by ammonia based beta-elimination.

Samples of glycoprotein, mixtures of glycoproteins, whole serum or other body fluids are reduced and alkylated by adding 4µl of 5X sample buffer (5X sample buffer: 0.625ml 0.5M Tris (6g for 100ml) adjusted to pH 6.6 with HCl, 1ml 10%SDS, 0.5ml glycerol, in 2.875ml water), 2µl of 0.5M dithiothreitol (DTT) and water to make up to 20µl in total, incubated at 70°C for 10min, then alkylated by addition of 2µl of 100mM iodoacetamide and incubated for 30 min in the dark at room temperature. Protein binding PVDF membranes (Durapore 13mm x 0.45 µm HVHP, Millipore) in Swinnex filter holders (Millipore) are pre-washed with 2 x 2.5 ml water using an all-polypropylene 2.5 ml syringe (Sigma), followed by a syringe full of air to remove most of the liquid from the membrane. The reduced and alkylated sample is then applied directly to the membrane and left to bind for 5 min before washing by pushing through 2 x 2.5 ml water slowly with a syringe, followed by a syringe full of air to remove most of the liquid from the membrane. The filter with the bound glycoprotein samples is then carefully removed from the filter holder and placed in a 1.5 ml screw capped polypropylene tube with a molded PTFE cap. 1 ml of ammonium carbonate saturated 29.2% aqueous ammonium hydroxide, plus 100mg ammonium carbonate is added to the tube. This is incubated for 40 hours at 60°C, then cooled in the fridge. The liquid is then transferred to a clean tube and evaporated to dryness. The released glycans are re-dissolved in water and re-dried until most of the salts are removed. 100 µl of 0.5M boric acid is added to the glycans and incubated at 37°C for 30 min. The glycans are then dried under vacuum, 1ml methanol added, re-dried, a further 1 ml methanol added and re-dried to remove the boric acid.

### Quantitatively analyzing the glycans.

### Labeling of glycans.

In some embodiments, upon releasing, the glycans can be labeled with, for example, a fluorescent label or a radioactive label. The fluorescent label can be, for example, 2-aminopyridine (2-AP), 2-aminobenzamide (2-AB), 2-aminoanthranilic acid (2-AA), 2-aminoacridone (AMAC) or 8-aminonaphthalene-1,3,6-trisulfonic acid (ANTS). Labeling of glycans with fluorescent labels is described, for example, by Bigge, J. C., et. al. "Nonselective and efficient fluorescent labeling of glycans using 2-amino benzamide and anthranilic acid." Anal Biochem 230: 229-38, 1995, and Anumula, K. R. (2000). High-sensitivity and high-resolution methods for glycoprotein analysis. Analytical Biochemistry 283: 17-26. Fluorescent labels can label all glycans efficiently and non-selectively and can enable detection and quantification of glycans in the sub picomole range. The choice of fluorescent label depends on the separation technique used. For example, a charged label is specifically required for capillary electrophoresis. In particular, 2-AB label can be preferred for chromatographic, enzymatic and mass spectroscopic processes and analyses, while 2-AA label can be preferred for electrophoretic analyses. Unlabelled glycans can be also detected by, for example, mass spectrometry, however, fluorescent labelling may aid glycan ionisation, see e.g. Harvey, D. J. (1999). "Matrix-assisted laser desorption/ionization mass spectrometry of carbohydrates." Mass Spectrom Rev 18: 349-450.; Harvey, D. J. (2000). Electrospray mass spectrometry and fragmentation of N-linked carbohydrates derivatized at the reducing terminus. J Am Soc Mass Spectrom 11: 900-915.

### Measuring glycoprofile of the released glycans.

Glycoprofile of the glycans means a presentation of particular glycan structures in the glycan pool. For example, when measured by HPLC, a glycoprofile can be a chromatogram comprising a plurality of peaks corresponding to glycan structures in the glycan pool. When measured by mass spectrometry, a glycoprofile can be a mass spectrum comprising a plurality of fragmentation patterns corresponding to glycan structures in the glycan pool. Measuring glycoprofile of the glycans can be carried out by quantitative analytical technique, such as chromatography, mass spectrometry, electrophoresis or a combination thereof. In particular, the chromatographic technique can be high performance anion exchange chromatography (HPAEC), weak ion exchange chromatography (WAX), gel permeation chromatography (GPC), high performance liquid chromatography (HPLC), normal phase high performance liquid chromatography (NP-HPLC), reverse phase HPLC (RP-HPLC), or porous graphite carbon HPLC (PGC-HPLC). The mass spectrometry technique can be, for example, matrix assisted laser desorption/ ionization time of flight mass spectrometry (MALDI-TOF-MS), electrospray ionization time of flight mass spectrometry (ESI-TOF-MS), positive or negative ion mass spectrometry or liquid chromatography mass spectrometry (LC-MS). The electrophoretic technique can be, for example, gel electrophoresis or capillary electrophoresis. The use of these quantitative analytical techniques for analyzing glycans is described, for example, in the following publications:
1) Guile, G. R., Wong, S. Y. and Dwek, R. A. (1994). "Analytical and preparative separation of anionic oligosaccharides by weak anion-exchange high-performance liquid chromatography on an inert polymer column." Analytical Biochemistry 222: 231-5 for HPLC;
2) Butler, M., Quelhas, D., Critchley, A. J., Carchon, H., Hebestreit, H. F., Hibbert, R. G., Vilarinho, L., Teles, E., Matthijs, G., Schollen, E., Argibay, P., Harvey, D. J., Dwek, R. A., Jaeken, J. and Rudd, P. M. (2003). "Detailed glycan analysis of serum glycoproteins of patients with congenital disorders of glycosylation indicates the specific defective glycan processing step and provides an insight into pathogenesis." Glycobiology 13: 601-22, for MALDI-MS, NP-HPLC and ESI-liquid chromatography/MS;
3) Jackson, P., Pluskal, M. G. and Skea, W. (1994). "The use of polyacrylamide gel electrophoresis for the analysis of acidic glycans labeled with the fluorophore 2-aminoacridone." Electrophoresis 15: 896-902, for polyacrylamide gel electrophoresis (PAGE);
4) Hardy, M. R. and Townsend, R. R. (1994). "High-pH anion-exchange chromatography of glycoprotein-derived carbohydrates." Methods Enzymol 230: 208-25., for HPAEC using pulsed amperometric detection (PAD);
5) Callewaert, N., Contreras, R., Mitnik-Gankin, L., Carey, L., Matsudaira, P. and Ehrlich, D. (2004). "Total serum protein N-glycome profiling on a capillary electrophoresis-microfluidics platform." Electrophoresis 25: 3128-31 for capillary electrophoresis;
6) Guile, G. R., Rudd, P. M., Wing, D. R., Prime, S. B. and Dwek, R. A. (1996). "A rapid high-resolution high-performance liquid chromatographic method for separating glycan mixtures and analyzing oligosaccharide profiles." Anal Biochem 240: 210-26, for HPLC;
7) Caesar, J. P., Jr., Sheeley, D. M. and Reinhold, V. N. (1990). "Femtomole oligosaccharide detection using a reducing-end derivative and chemical ionization mass spectrometry." Anal Biochem 191: 247-52, for LC-MS;
8) Mattu, T. S., Royle, L., Langridge, J., Wormald, M. R., Van den Steen, P. E., Van Damme, J., Opdenakker, G., Harvey, D. J., Dwek, R. A. and Rudd, P. M. (2000). "O-glycan analysis of natural human neutrophil gelatinase B using a combination of normal phase-HPLC and online tandem mass spectrometry: implications for the domain organization of the enzyme." Biochemistry 39: 15695-704, for NP-HPLC and MS;
9) Royle, L., Mattu, T. S., Hart, E., Langridge, J. I., Merry, A. H., Murphy, N., Harvey, D. J., Dwek, R. A. and Rudd, P. M. (2002). "An analytical and structural database provides a strategy for sequencing O-glycans from microgram quantities of glycoproteins." Anal Biochem 304: 70-90, for NP-HPLC and MS;
10) Anumula, K. R. and Du, P. (1999). "Characterization of carbohydrates using highly fluorescent 2- aminobenzoic acid tag following gel electrophoresis of glycoproteins." Anal Biochem 275: 236-42, for gel electrophoresis;
11) Huang, Y. and Mechref, Y. (2001). "Microscale nonreductive release of O-linked glycans for subsequent analysis through MALDI mass spectrometry and capillary electrophoresis." Analytical Chemistry 73: 6063-6069, for a combination of MALDI-MS and capillary electrophoresis;
12) Burlingame, A. L. (1996). "Characterization of protein glycosylation by mass spectrometry." Curr Opin Biotechnol 7: 4-10, for mass spectrometry;
13) Costello, C. E. (1999). "Bioanalytic applications of mass spectrometry." Curr Opin Biotechnol 10: 22-8, for mass spectrometry;
14) Davies, M. J. and Hounsell, E. F. (1996). "Comparison of separation modes of high-performance liquid chromatography for the analysis of glycoprotein- and proteoglycan-derived oligosaccharides." J Chromatogr A 720: 227-33, for HPLC;
15) El Rassi, Z. (1999). "Recent developments in capillary electrophoresis and capillary electrochromatography of carbohydrate species." Electrophoresis 20: 3134-44, for capillary electrophoresis and capillary electrochromatography;
16) Kuster, B., Wheeler, S. F., Hunter, A. P., Dwek, R. A. and Harvey, D. J. (1997). "Sequencing of N-linked oligosaccharides directly from protein gels: in-gel deglycosylation followed by matrix-assisted laser desorption/ionization mass spectrometry and normal-phase high-performance liquid chromatography." Anal-Biochem 250: 82-101, for NP-HPLC and MALDI-MS;
17) Reinhold, V. N., Reinhold, B. B. and Chan, S. (1996). "Carbohydrate sequence analysis by electrospray ionization-mass spectrometry." Methods Enzymol 271: 377-402, for ESI-MS;
18) Mattu, T. S., Pleass, R. J., Willis, A. C., Kilian, M., Wormald, M. R., Lellouch, A. C., Rudd, P. M., Woof, J. M. and Dwek, R. A. (1998). "The glycosylation and structure of human serum IgA1, Fab, and Fc regions and the role of N-glycosylation on Fc alpha receptor interactions." Journal of Biological Chemistry 273: 2260-72, for WAX and NP-HPLC;
19) Callewaert, N., Schollen, E., Vanhecke, A., Jaeken, J., Matthijs, G., and Contreras, R. (2003). Increased fucosylation and reduced branching of serum glycoprotein N-glycans in all known subtypes of congenital disorder of glycosylation I. Glycobiology 13: 367-375;
20) Block, T.M. Comunale, M.A., Lowman, M., Steel, L.F., Romano, P.R." Fimmel, C., Tennant, B.C. London, A.A. Evans, B.S. Blumberg, R.A. Dwek, T.S. Mattu and A.S. Mehta , "Use of targeted glycoproteomics to identify serum glycoproteins that correlate with liver cancer in woodchucks and humans." PNAS USA (2005) 102, 779-784;
21) D. J. Harvey, Fragmentation of negative ions from carbohydrates: Part 1; Use of nitrate and other anionic adducts for the production of negative ion electrospray spectra from N-linked carbohydrates, J. Am. Soc. Mass Spectrom., 2005, 16, 622-630;
22) D. J. Harvey, Fragmentation of negative ions from carbohydrates: Part 2, Fragmentation of high-mannose N-linked glycans, J. Am. Soc. Mass Spectrom., 2005, 16, 631-646;
23) D. J. Harvey, Fragmentation of negative ions from carbohydrates: Part 3, Fragmentation of hybrid and complex N-linked glycans, J. Am. Soc. Mass Spectrom., 2005, 16, 647-659.

Although many techniques can be used for measuring glycoprofile, in the method of determining one or more glycosylation markers of disease, it can be preferred to measure glycoprofiles by high performance liquid chromatography (HPLC) alone or in combination with mass spectrometry. For example, measuring glycoprofiles can be performed by gel electrophoresis (see Jackson, P., Pluskal, M. G. and Skea, W. (1994). "The use of polyacrylamide gel electrophoresis for the analysis of acidic glycans labeled with the fluorophore 2-aminoacridone." Electrophoresis 15: 896-902); HPAEC using pulsed amperometric detection (PAD) (Townsend, R. R., Hardy, M. R., Hindsgaul, O. and Lee, Y. C. (1988). "High-performance anion-exchange chromatography of oligosaccharides using pellicular resins and pulsed amperometric detection." Anal Biochem 174: 459-70; and Hardy, M. R. and Townsend, R. R. (1994). "High-pH anion-exchange chromatography of glycoprotein-derived carbohydrates." Methods Enzymol 230: 208-25);or capillary electrophoresis (see El Rassi, Z. (1999). "Recent developments in capillary electrophoresis and capillary electrochromatography of carbohydrate species." Electrophoresis 20: 3134-44), however, these techniques are not ideally suited to large-scale automation, nor do they provide full quantitative structural analysis. In general they have poor detection limits, low reproducibility and are restricted by the inherent difficulty of obtaining full structural characterization of the oligosaccharides and the lack of predictability that is required to enable the preliminary assignments to be made to novel structures.

Measuring glycoprofiles of fluorescently labeled glycans (e.g. 2AB labeled glycans) by HPLC can allow accurate quantification and structural assignment of the glycan structures in the glycan pool by integration of the peaks in the chromatogram. For HPLC measured glycoprofile, glycan structures present in the analyzed glycan pool are separated based on their elution time. For NP-HPLC, the elution times can be converted to glucose units by comparison with a standard dextran hydrolysate ladder. HPLC measured glycoprofile can trace all glycan structures present in a glycan pool in correct molar proportions. Polar functional groups of stationary phase of HPLC can interact with the hydroxyl groups of the glycans in a manner that is reproducible for a particular monosaccharide linked in a specific manner. For example, the contribution of the outer arm fucose addition is much greater than the addition of a core fucose residue; a core fucose residue always contributes 0.5 glucose units (gu) to the overall elution position. The characteristic incremental values associated with different monosaccharide additions can allow the preliminary assignment of a predicted structure for a particular peak present in the glycoprofile. This structure can be then confirmed by digestion with exoglycosidase arrays and/or mass spectrometry. Other techniques, such as capillary electrophoresis are not as predictable as NP-HPLC. Although, CE migration times can be calibrated with standards, the migration times of unknown structures can not be easily predicted. Measuring glycoprofiles by NP-HPLC can be also preferred for the following reason. Digestion of a glycan pool with one or more exoglycosidases removes monosaccharide residues and, thus, decreases the retention times or associated gu values in the glycoprofile measured by NP-HPLC. In some embodiments, this can enable the segregation of the peaks that are associated with one or glycosylation markers by shifting away peaks that are not related to the glycosylation changes away from the measured region of the glycoprofile.

In some embodiments, measuring glycoprofiles can be carried out using reverse phase high performance liquid chromatography. For RP-HPLC measured glycoprofiles, the elution times can be converted into arabinose units using a standard arabinose ladder. The use of RP-HPLC for measuring glycosylation profiles is described, for example, in Guile, G. R., Harvey, D. J., O'Donnell, N., Powell, A. K., Hunter, A. P., Zamze, S., Fernandes, D. L., Dwek, R. A., and Wing, D. R. (1998). "Identification of highly fucosylated N-linked oligosaccharides from the human parotid gland. European Journal of Biochemistry" 258: 623-656; Royle, L., Mattu, T. S., Hart, E., Langridge, J. I., Merry, A. H., Murphy, N., Harvey, D. J., Dwek, R. A., and Rudd, P. M. (2002). An analytical and structural database provides a strategy for sequencing o-glycans from microgram quantities of glycoproteins. Analytical Biochemistry 304: 70-90. RP-BPLC measured glycoprofiles can be used to complement glycoprofiles measured by NP-HPLC. For example, RP-HPLC can separate bisected glycan structures from glycan structures that do not comprise bisecting *N*-acetylglucoamine residue. In NP-HPLC measured glycoprofiles these structures can be too close to be resolved. In some embodiments, measuring glycoprofiles by RP-HPLC can comprise using one or more buffers. The mobile phase can be for example, to improve the reproducibility of the measurement. The buffer can be, for example, solvent A: 50mM of ammonium formate adjusted to pH5 with triethylamine and solvent B: solvent A and acetonitrile mixed 50/50.

In some embodiments, HPLC can be used as a preparative method for collecting glycans, i.e. HPLC can be used to isolate unusual glycans for further analysis, by e.g. mass spectrometry, as well as for obtaining parameters for a glycan database.

In some embodiments, each of the glycoprofiles can be presented as a plurality of peaks corresponding to glycan structures in the glycans. In the method of determining one or more glycosylation markers, a peak ratio means a ratio between any one or more peaks and any other one or more peaks within the same glycosylation profile. In the method of determining a glycosylation marker, comparing peak ratios can mean comparing peaks intensities or comparing integrated areas under the peaks. In some embodiments of the method of determining glycosylation marker, comparing peak ratios can be carried for glycans of the diseased and control samples which were not digested with one or more exoglycosidases. In some embodiments, comparing peak ratios can be carried out on the glycans which were digested with one or more exoglycosidases. In some embodiments, comparing peak ratios can be carried out for the glycans which were not digested with exoglycosidase and for the glycans digested with one or more exoglycosidases.

In some embodiments, measuring glycoprofiles with HPLC can be complemented with a mass spectrometry measurement. Complementary mass spectrometry data, such as MALDI, ESI or LC/MS) can serve, for example, for validation HPLC measured glycoprofiles as a separate orthogonal technique able to resolve the structures of more complex glycans when a sufficient amount of sample of a body fluid or a body tissue is available. Mass spectrometry used in combination with HPLC can be a powerful tool for structural analysis of glycoproteins. Mass spectrometry alone can be used for structural analysis of glycans providing monosaccharide composition of glycans. However, mass spectrometry used by itself does not distinguish isobaric monosaccharide (and hence oligosaccharides or glycans) and does not provide the information on monosaccharide linkage in glycans. The LC-MS/(MS) techniques can provide the most informative data out of the mass spectrometry technique, see Caesar, J. P., Jr., Sheeley, D. M. and Reinhold, V. N. (1990). "Femtomole oligosaccharide detection using a reducing-end derivative and chemical ionization mass spectrometry." Anal Biochem 191: 247-52; Mattu, T. S., Pleass, R. J., Willis, A. C., Kilian, M., Wormald, M. R., Lellouch, A. C., Rudd, P. M., Woof, J. M. and Dwek, R. A. (1998). "The glycosylation and structure of human serum IgA1, Fab, and Fc regions and the role of N-glycosylation on Fc alpha receptor interactions." Journal of Biological Chemistry 273: 2260-72; and Royle, L., Mattu, T. S., Hart, E., Langridge, J. I., Merry, A. H., Murphy, N., Harvey, D. J., Dwek, R. A. and Rudd, P. M. (2002). "An analytical and structural database provides a strategy for sequencing O-glycans from microgram quantities of glycoproteins." Anal Biochem 304: 70-90. In some embodiments, measuring glycoprofiles by LC/MS can comprise using the LC stage of LC/MS not only for cleanup and preliminary separation of glycans before they enter the MS stage of LC/MS but for obtaining preliminary assignment of glycan structures in the glycans. This can be accomplished, for example, by using NP-HPLC matrix, for example NP-HPLC with TSK gel amide 80 matrix, in the LC column of LC/MS. In NP-HPLC with TSK gel amide 80 matrix, hydroxyl groups of glycans interact with the amide functionality, therefore, the elution order is determined by the number of hydroxyl groups in a particular glycan, its molecular confirmation and its relative solubility in the mobile phase. As a first line approach, especially when amounts of body fluid or body tissue are limited, mass spectrometry may not be sufficient by itself for accurate quantitative characterization and full structural assignment including linkages and monosaccharide sequence of glycan structures in the pool of glycans.

In some embodiments of the invention, when the glycan pool comprises charged glycans, the glycan pool can be fractioned into several aliquots based upon charge. Fractioning of the glycan pool can be carried out, for example, by weak ion exchange (WAX) chromatography. Each WAX aliquot can be then analyzed independently by NP-HPLC combined with exoglycosidase digestions. Measuring glycoprofiles by WAX HPLC is described, for example, in Guile, G. R., Wong, S. Y. and Dwek, R. A. (1994). "Analytical and preparative separation of anionic oligosaccharides by weak anion-exchange high-performance liquid chromatography on an inert polymer column." Analytical Biochemistry 222: 231-5.

Measuring glycoprofile of the glycans with the above described methods can allow to detect a particular glycan structure present in the glycans in subpicomole levels. Accordingly, in some of the embodiments, measuring glycoprofiles of the glycans is carried out using a technique able to detect a glycan structure present in the glycans in amount of 1 picomole, preferably 0.1 picomole, yet more preferably 0.01 picomole.

### Exoglycosidase digestion

In some embodiments, the released glycans can be subjected to further enzymatic digestion with one or more enzymes. The enzymatic digestion can be done using any suitable enzymes, such as glycosidases. Examples of suitable glycosidases include, but are not limited to, N-glycosidase F (PNGase F), sialidase, β-galactosidase, fucosidase α1-6,2>>3,4, α1-3,4, α1-2 fucosidase, alpha-amylase, beta-amylase, glucan 1,4-alpha-glucosidase, cellulase, endo-1,3(4)-beta-glucanase, inulinase, endo-1,4-beta-xylanase, oligosaccharide alpha-1,6-glucosidase, dextranase, chitinase, polygalacturonase, lysozyme, exo-alpha-sialidase, alpha-glucosidase, beta-glucosidase, alpha-galactosidase, beta-galactosidase, alpha-mannosidase, beta-mannosidase, beta-fructofuranosidase, alpha,alpha-trehalase, beta-glucuronidase, xylan endo-1,3-beta-xylosidase, amylo-alpha-1,6-glucosidase, hyaluronoglucosaminidase, hyaluronoglucuronidase, xylan 1,4-beta-xylosidase, beta-D-fucosidase, glucan endo-1,3-beta-D-glucosidase, alpha-L-rhamnosidase, pullulanase, GDP-glucosidase, beta-L-rhamnosidase, fucoidanase, glucosylceramidase, galactosylceramidase, galactosylgalactosylglucosylceramidase, sucrose alpha-glucosidase, alpha-N-acetylgalactosaminidase, alpha-N-acetylglucosaminidase, alpha-L-fucosidase, beta-N-acetylhexosaminidase, beta-N-acetylgalactosaminidase, cyclomaltodextrinase, alpha-L-arabinofuranosidase, glucuronosyl-disulfoglucosamine glucuronidase, isopullulanase, glucan 1,3-beta-glucosidase, glucan endo-1,3-alpha-glucosidase, glucan 1,4-alpha-maltotetrahydrolase, mycodextranase, glycosylceramidase, 1,2-alpha-L-fucosidase, 2,6-beta-fructan 6-levanbiohydrolase, levanase, quercitrinase, galacturan 1,4-alpha-galacturonidase, isoamylase, glucan 1,6-alpha-glucosidase, glucan endo-1,2-beta-glucosidase, xylan 1,3-beta-xylosidase, licheninase, glucan 1,4-beta-glucosidase, glucan endo-1,6-beta-glucosidase, L-iduronidase, mannan 1,2-(1,3)-alpha-mannosidase, mannan endo-1,4-beta-mannosidase, fructan beta-fructosidase, agarase, exo-poly-alpha-galacturonosidase, kappa-carrageenase, glucan 1,3-alpha-glucosidase, 6-phospho-beta-galactosidase, 6-phospho-beta-glucosidase, capsular-polysaccharide endo-1,3-alpha-galactosidase, beta-L-arabinosidase, arabinogalactan endo-1,4-beta-galactosidase, cellulose 1,4-beta-cellobiosidase, peptidoglycan beta-N-acetyhnuramidase, alpha,alpha-phosphotrehalase, glucan 1,6-alpha-isomaltosidase, dextran 1,6-alpha-isomaltotriosidase, mannosyl-glycoprotein endo-beta-N-acetylglucosamidase, glycopeptide alpha-N-acetylgalactosaminidase, glucan 1,4-alpha-maltohexaosidase, arabinan endo-1,5-alpha-L-arabinosidase, mannan 1,4-beta-mannobiosidase, mannan endo-1,6-beta-mannosidase, blood-group-substance endo-1,4-beta-galactosidase, keratan-sulfate endo-1,4-beta-galactosidase, steryl-beta-glucosidase, strictosidine beta-glucosidase, mannosyl-oligosaccharide glucosidase, protein-glucosylgalactosylhydroxylysine glucosidase, lactase, endogalactosaminidase, mucinaminylserine mucinaminidase, 1,3-alpha-L-fucosidase, 2-deoxyglucosidase, mannosyl-oligosaccharide 1,2-alpha-mannosidase, mannosyl-oligosaccharide 1,3-1,6-alpha-mannosidase, branched-dextran exo-1,2-alpha-glucosidase, glucan 1,4-alpha-maltotriohydrolase, amygdalin beta-glucosidase, prunasin beta-glucosidase, vicianin beta-glucosidase, oligoxyloglucan beta-glycosidase, polymannuronate hydrolase, maltose-6'-phosphate glucosidase, endoglycosylceramidase, 3-deoxy-2-octulosonidase, raucaffricine beta-glucosidase, coniferin beta-glucosidase, 1,6-alpha-L-fucosidase, glycyrrhizinate beta-glucuronidase, endo-alpha-sialidase, glycoprotein endo-alpha-1,2-mannosidase, xylan alpha-1,2-glucuronosidase, chitosanase, glucan 1,4-alpha-maltohydrolase, difructose-anhydride synthase, neopullulanase, glucuronoarabinoxylan endo-1,4-beta-xylanase, mannan exo-1,2-1,6-alpha-mannosidase, anhydrosialidase, alpha-glucosiduronase, lacto-N-biosidase, 4-alpha-D-{(1->4)-alpha-D-glucano}trehalose trehalohydrolase, limit dextrinase, poly(ADP-ribose) glycohydrolase, 3-deoxyoctulosonase, galactan 1,3-beta-galactosidase, beta-galactofuranosidase, thioglucosidase, ribosylhomocysteinase, beta-primeverosidase.

Most preferably, enzymatic digestion is carried out with one or more exoglycosidases listed in table 1.

| **Table 1 - Exoglycosidase Specificities -** | |
|---|---|
| Sialidase α-(2-3,6,8) | Cleaves all non-reducing terminal branched and unbranched sialic acids |
| Sialidase α-(2-3) | Cleaves the non-reducing terminal alpha-(2-3) unbranched sialic acid residues from complex carbohydrates and glycoproteins. |
| α-(1-3,4,6)-galactosidase | Cleaves α-(1-3)-, α-(1-4)-and α-(1-6)-linked, non-reducing terminal galactose from complex carbohydrates and glycoproteins. Fucose linked to the penultimate *N*-acetylglucosamine will block cleavage of the galactose. |
| β-(1-4)-galactosidase | Cleaves Non-reducing terminal β-(1-4)-galactose. Fucose linked to the penultimate *N*-acetylglucosamine will block cleavage of the galactose. |
| β-(1-3,4,6)-galactosidase | Cleaves all β1-3 and β1-4 linked non-reducing, terminal galactose. Fucose linked to the penultimate *N*-acetylglucosamine will block cleavage of the galactose. |
| β-(1-3,6)-galactosidase | Cleaves β-(1-3)- and β-(1-6)-linked, non-reducing terminal galactose from complex carbohydrates and glycoproteins. Fucose, but not sialic acid, linked to the penultimate N-acetylglucosamine will block cleavage. |
| β-*N*-acetylglucosaminidase | Cleaves all non-reducing terminal β-linked *N*-acetylglucosamine. Bisecting GlcNAc slows the reaction. |
| β-*N*-acetylhexosaminidase | Cleaves all non-reducing terminal β-linked *N*-acetylglucosamine and *N*-acetylgalacosamine. Bisecting GlcNAc slows the reaction |
| α-(1-2,3,6)-mannosidase | Cleaves all α-(1-2,3,6)-linked mannose. |
| α-(1-6)-core mannosidase | Cleaves unbranched, terminal non-reducing mannose linked alpha-(1-6) to the mannosyl chitobiose core. The presence of a branched mannose alpha-(1-3) will inhibit the removal of the 1-6 mannose. |
| α-(1-3,4)-fucosidase | Cleaves non-reducing terminal branched fucose when linked alpha-(1-3) or alpha-(1-4) to GlcNAc. |
| α-(1-6>2>>3,4)-fucosidase | Cleaves non-reducing terminal branched fucose when linked alpha-(1-6) to GlcNAc or Gal. Will also cleave alpha-(1-2) and alpha-(1-3,4) with reduced efficiency |
| α-(1-2)-fucosidase | Cleaves non-reducing terminal branched fucose when linked alpha-(1-2) to a Gal. |

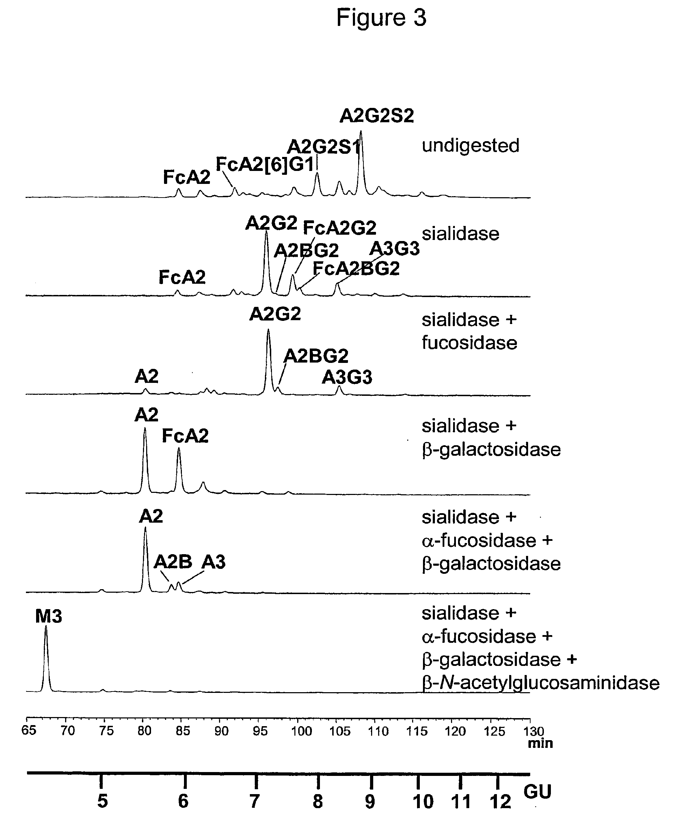

For example, figure 3 illustrates NP-HPLC measured glycoprofiles of 2-AB labeled *N*-linked glycans released from whole serum after following digestion with an array of exoglycosidases. Only major peaks are annotated on figure 3, abbreviations used correspond to Table 2.

In some embodiments, the enzymatic digestion can be sequential, so not all monosaccharides are removed at once. The digested glycans can be analyzed after each digestion step to obtain a glycosylation profile. In some embodiments, the enzymatic digestion can be digestion with an array comprising one or more exoglycosidases. Digestion with an array means using a panel of exoglycosidases in various combinations to provide several digestion profiles on aliquots of a pool of glycans. Each exoglycosidase enzyme removes specific terminal monosaccharides attached in defined linkages. In an array, the exoglycosidase enzymes act sequentially depending on their specificity.

In some embodiments of the invention, digestion with one or more exoglycosidases in any combination can be used to segregate the glycosylation marker(s) by shifting glycan structures that do not contain the marker from the measured region of the glycoprofile. In some embodiments of the invention, digestion with one or more exoglycosidases in any combination can be used to *amplify* the glycosylation marker(s) by digesting away monosaccharides that are attached to some of the markers oligosaccharides but are not essential feature of the markers. In some embodiments of the invention, digestion with one or more exoglycosidases can be used to both amplify and segregate the glycosylation marker(s). The use of digestion with one or more exoglycosidases to segregate and/or amplify glycosylation markers is illustrated, for example, in the US provisional application "Glycosylation Markers for Cancer Diagnostics and Monitoring" to Dwek *et. al*. filed , incorporated hereby by reference.

In some embodiments, measuring glycoprofiles can reveal one or more peaks which can not be assigned to previously reported glycan structures or structures that are not fully digested by the enzyme array panels. In this case, the digestion with one or more exoglycosidases can be used to segregate these one or peaks for further analysis. The HPLC based technology allows such glycans to be recovered from the HPLC eluate for further analysis.

### Databases.

Measuring glycoprofile of the glycans can comprise constructing a database of glycan structures of the glycans. The parameters of this database can be, for example, glycan structure along with: elution times (from HPLC data); mass and composition (from MS data); experimentally determined and/or predicted glycan structures, elution times, mass and composition, following treatment with exoglycosidase enzymes; experimentally determined and/or predicted glycan structures, mass and composition following MS fragmentation. The database can, for example, make preliminary and final assignments of the glycan structures as well as recommend the appropriate exoglycosidase arrays to confirm preliminary assignments. The use of databases in measuring glycoprofiles is described, for example, in the following references:
1) Mattu, T. S., Royle, L., Langridge, J., Wormald, M. R., Van den Steen, P. E., Van Damme, J., Opdenakker, G., Harvey, D. H., Dwek, R. A. and Rudd, P. M. (2000). "The O-glycan analysis of natural human neutrophil gelatinase B using a novel strategy combining normal phase- HPLC and on-line tandem mass spectrometry: implications for the domain organization of the enzyme." Biochemistry 39: 15695-704.,";
2) Royle, L., Mattu, T. S., Hart, E., Langridge, J. I., Merry, A. H., Murphy, N., Harvey, D. J., Dwek, R. A. and Rudd, P. M. (2002). "An analytical and structural database provides a strategy for sequencing O-glycans from microgram quantities of glycoproteins." Anal Biochem 304: 70-90,
3) Butler, M., Quelhas, D., Critchley, A. J., Carchon, H., Hebestreit, H. F., Hibbert, R. G., Vilarinho, L., Teles, E., Matthijs, G., Schollen, E., Argibay, P., Harvey, D. J., Dwek, R. A., Jaeken, J. and Rudd, P. M. (2003). "Detailed glycan analysis of serum glycoproteins of patients with congenital disorders of glycosylation indicates the specific defective glycan processing step and provides an insight into pathogenesis." Glycobiology 13: 601-22,
4) Peracaula, R., Royle, L., Tabares, G., Mallorqui-Fernandez, G., Barrabes, S., Harvey, D. J., Dwek, R. A., Rudd, P. M. and de Llorens, R. (2003). "Glycosylation of human pancreatic ribonuclease: differences between normal and tumor states." Glycobiology 13: 227-44,
5) Peracaula, R., Tabares, G., Royle, L., Harvey, D. J., Dwek, R. A., Rudd, P. M. and de Llorens, R. (2003). "Altered glycosylation pattern allows the distinction between prostate-specific antigen (PSA) from normal and tumor origins." Glycobiology 13: 457-70.

One example of the glycan database can be a database comprising glycan structures determined by negative ion mass spectrometry at Oxford Glycobilogy Institute. The use of negative ion mass spectrometry for glycan analysis is described, for example, in
1) D. J. Harvey, Fragmentation of negative ions from carbohydrates: Part 1; Use of nitrate and other anionic adducts for the production of negative ion electrospray spectra from N-linked carbohydrates, J. Am. Soc. Mass Spectrom., 2005, 16, 622-630,
2) D. J. Harvey, Fragmentation of negative ions from carbohydrates: Part 2, Fragmentation of high-mannose N-linked glycans, J. Am. Soc. Mass Spectrom., 2005, 16, 631-646,
3) D. J. Harvey, Fragmentation of negative ions from carbohydrates: Part 3, Fragmentation of hybrid and complex N-linked glyeans, J. Am. Soc. Mass Spectrom., 2005, 16, 647-659.

The database currently includes fragmentation patterns of 60 glycan structures from the mass spectrometric profiles of individual oligosaccharides and of mixtures of oligosaccharides. The patterns of this database can be matched to experimental MS profiles by pattern matching using commercially available software.

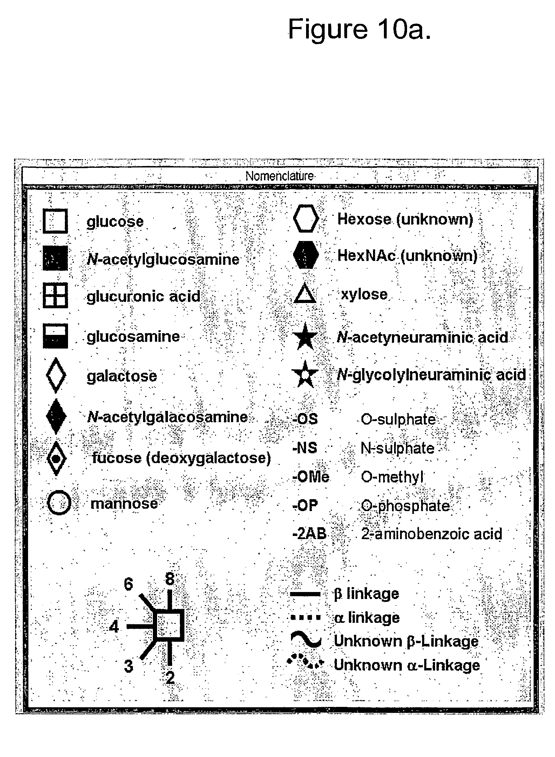

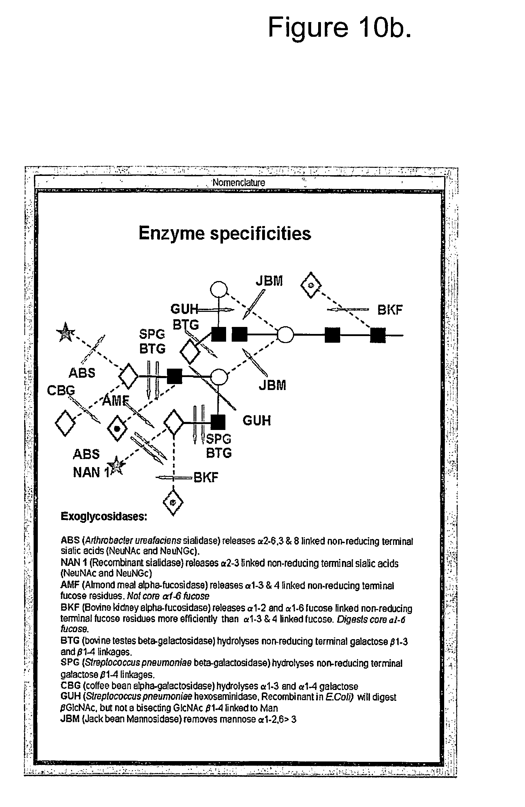

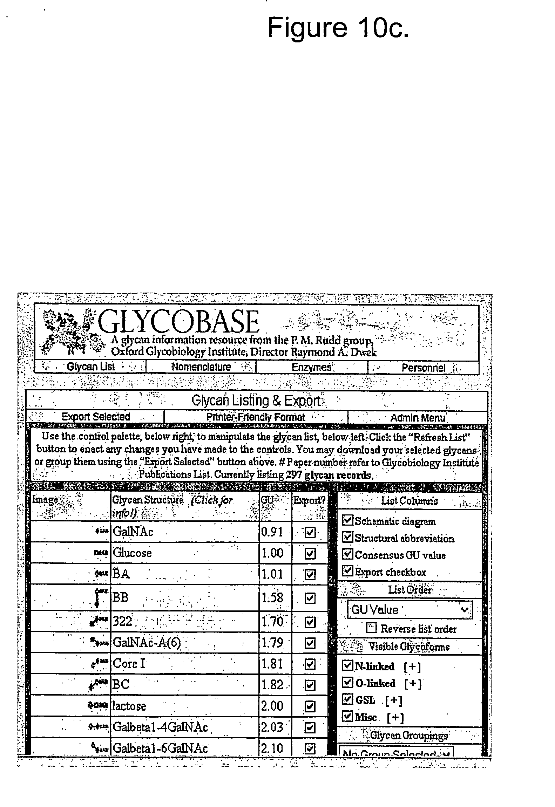

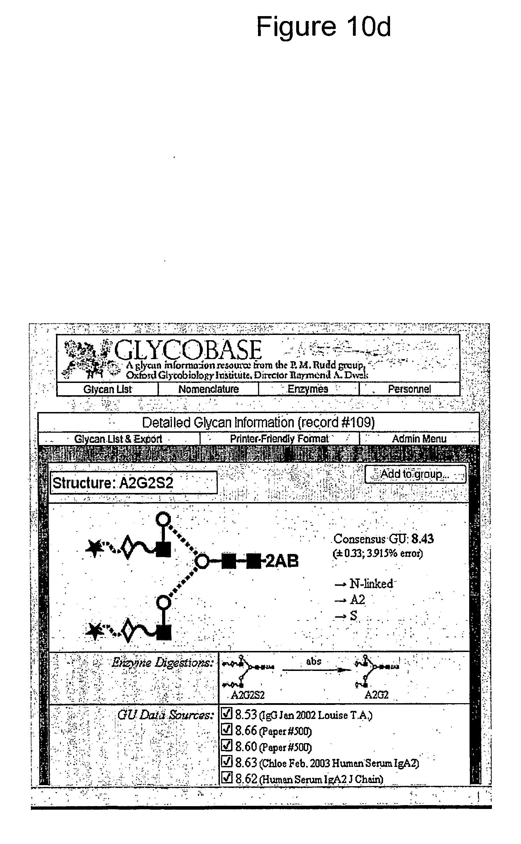

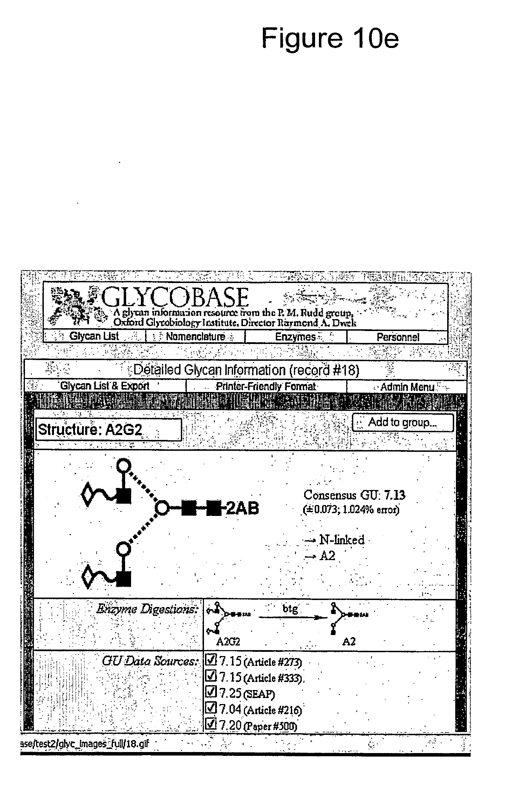

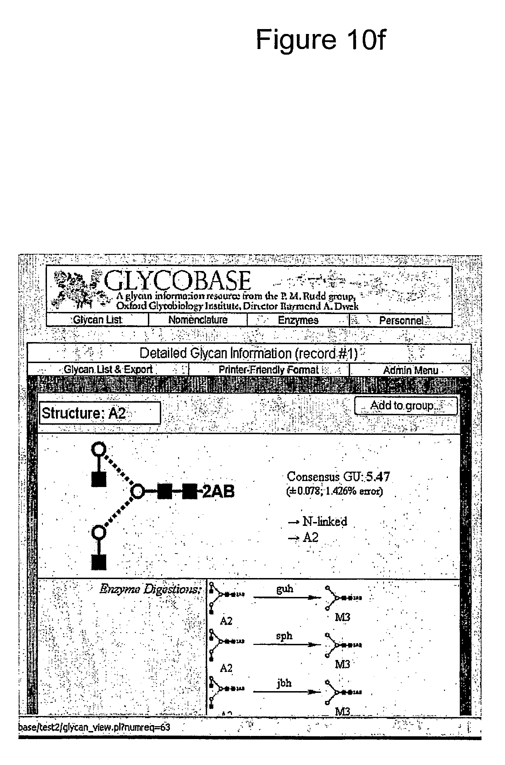

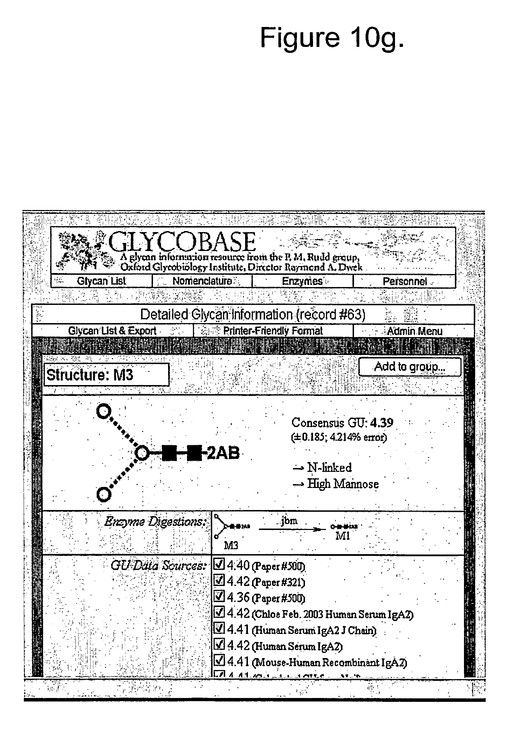

The example of the glycan database can be Glycobase, a glycan database at Oxford Glycobiology Institute (OGBI) illustrated on Figure 10. Glycobase contains analytical data for over 290 glycan structures and can be used to assign both preliminary and final structures as well as to recommend the appropriate exoglycosidase arrays to confirm preliminary assignments as illustrated in Figure 10. Panels (a) & (b) of figure 10 show the nomenclature that can be used to draw the glycan structures and explain the exoglycosidase digestions. Panel (c) shows some of the structures with GU values listed in the Glycobase; panels (d) to (g) follow the glycan structure A2G2S2 (the abbreviations used are explained in Table 2) thorough a series of digestions with exoglycosidases, in each case the consensus GU value (which is calculated from the experimentally determined GU values listed) is given along with possible digestions and products.

GU values for individual peaks can be generated, for example, using the PeakTime add on software package. The PeakTime software automatically can calculate, for example, the GU value for each sample peak based upon the comparison with dextran ladder standard and can list preliminary assignments to each peak using data from the standard database. An additional software, like PeakShift at OGBI, can be used to assign structures to the products of enzyme assign structures to the products of enzyme digests and confirm which of the initial assignments, made by PeakTime, is correct. The Peakshift software uses a combination of peak areas and the incremental values for individual monosaccharide residues.

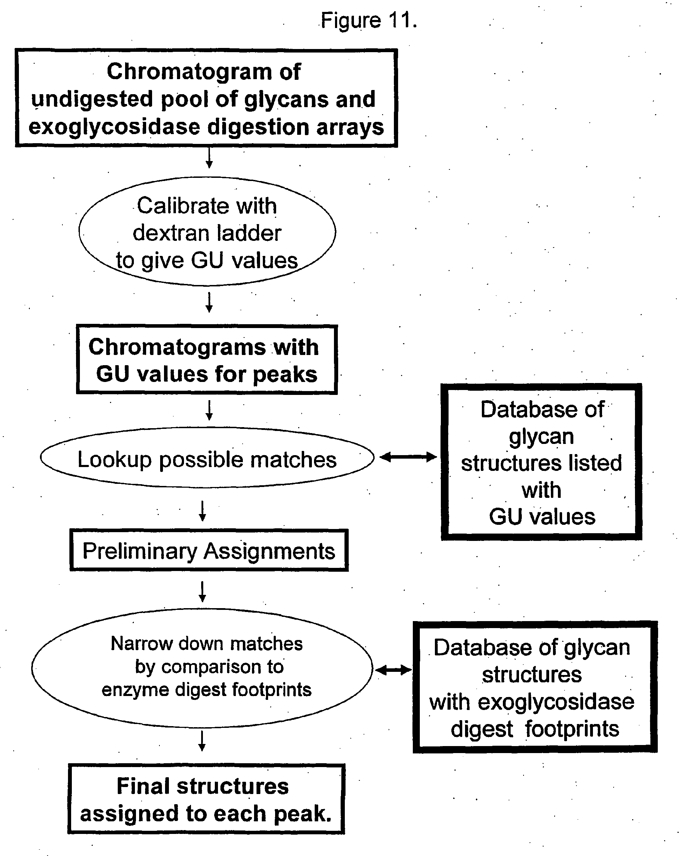

An algorithm (figure 11) based on a model of generalized transformations on a set of chromatogram peaks with a given variance can be applied for further analyzing glycosylation profiles. In the field of glycobiology, these transformations may represent the action of enzymes, e.g. exoglycosidases; however the generality of the algorithm lends its applicability outside of the field to any area of chemistry or biochemistry in which similar methods are used. The algorithm can be resolved into components. Firstly, each chromatogram can be calibrated on a series of standard peaks using a polynomial curve fitting technique. A first list of possible assignments can be then determined for each peak by table lookup against a database of standard values. A statistically valid procedure can be used in the comparison, employing the known variance of both unknowns and standards. The final step can be a comparison with the stored enzyme digest footprints of the standards.

The database currently in use at OGBI is continually updated as new data are acquired. The database can display the structural abbreviation, schematic diagram, consensus GU value (having calculated an average from the data entered for that structure) and digestion products (which are entered for a range of exoglycosidase digestions). The subsections of the database can be N-links animal; N-links plants; N-links high mannose; O-links core 1&2; O-links core 3&4; O-links other; GSL; and miscellaneous. Further modifications of the database can enable a larger range of subsections to be chosen. The database can potentially allow the user to choose which glycans to view by choices such as: which sugar (e.g. fucose) they contain; or all N-glycan biantennary structures only.

### Serum glycome data base

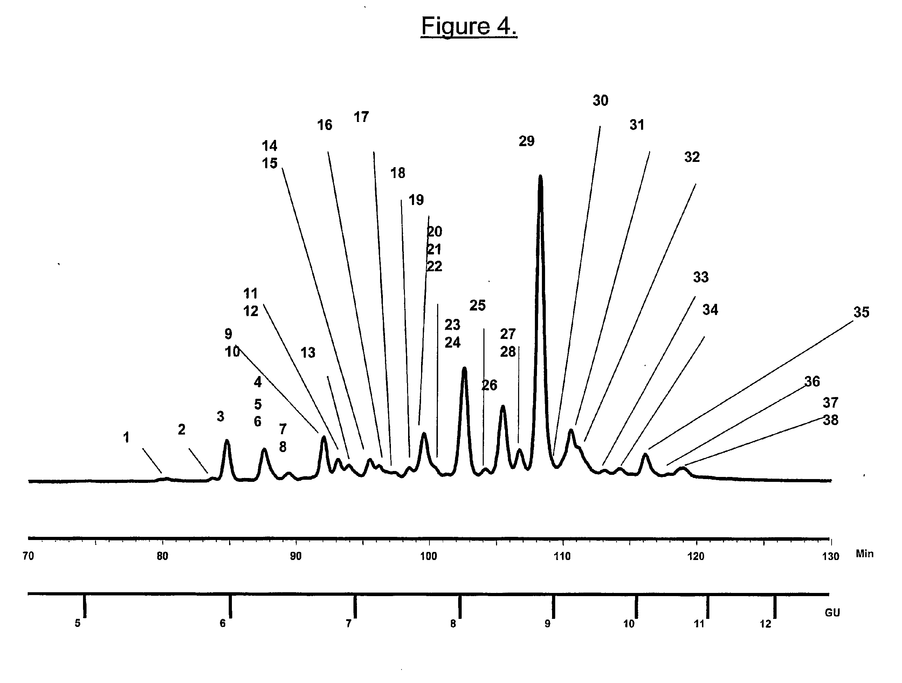

Separate database can be constructed for glycans released for whole serum without purifying the glycoproteins. For example, a specific database can be constructed containing NP-HPLC serum glycan profiles for both sialylated and neutral glycans with currently 38 glycans identified on Fig. 4 and in Table 2.

*The following functionality has been completed, fully tested and is currently in use in the laboratory at the Oxford Glycobiology Institute*:
1) A database which holds the specificities of enzymes and the standard GU values and variances. The structure of the database is flexible one, allowing different values to be stored for different columns and methods and allowing the store to be divided easily into areas for different chemical groups, or different users or projects.
2) Graphical display. The latest version of PeakTime is able to display multiple plots one above the other to the same scale, and to show the transformations due to enzymes as joins amongst their peaks.
3) The chromatogram may be drawn with the time axis recalculated to the calibrated scale. This allows direct comparisons to be made between multiple plots, especially as values from the database can be displayed as stick-graphs on the same scale.
4) The graphical functions provided include zooming-in, displaying as a stick-graph or the raw curve, switching between peak areas and heights, and annotating with such information as the chemical species name determined from sequencing.
5) Tabular display. Most of what is shown graphically can also be displayed in tabular form. The tables may be adjusted in the width and height of cells, and individual columns may be hidden in a manner that will be familiar to users of spreadsheets.
6) A security system of login names and passwords is provided, allowing the standard data to be protected against unauthorized alteration, and for individual users to restrict access to their private data.
7) A column calibration facility with a polynomial mapping. The range of different calibration types performed includes both the glucose units (GU) and the arabinose units (AU) scales.
8) Operations on chromatograms. A sum and a difference function are provided. Such plots would be hard to interpret without the calibration that PeakTime performs.
9) A rapid data entry mechanism by which experimental data may be added to the database by drag-and-drop with the mouse directly from a chromatogram. This is designed to encourage use of the database for ephemeral day-to-day values, and so to reduce dependence on paper note keeping.
10) Locking. Once calibrated the chromatogram can be locked so that inadvertent alterations may be avoided
11) Export. Graphics may be exported to files in either bitmap or vector format, or as bitmaps to the windows clipboard. All tables can be exported to files in standard formats for transfer to such tools as Microsoft Access or Excel.
12) Printing. Both graphical and tabular displays can be printed from within PeakTime, and in some cases a print-preview facility is provided. Various display choices are provided, such as fonts, line thicknesses and column widths.

### Using the glycosylation markers of disease to identify and isolate glycoproteins

In some embodiments, the determined glycosylation marker of disease can be used for identifying and isolating one or more glycoprotein biomarkers, i.e. glycoproteins that are specific for the disease. The glycoprotein biomarker of the disease carries the glycosylation marker of the disease. The isolation of the glycoprotein biomarkers of the disease can be carried out using lectins or monoclonal antibodies. For example, lectins were used to isolate gp73, a glycoprotein marker of hepatitis B associated with liver cancer in "Use of targeted glycoproteomics to identify serum glycoproteins that correlate with liver cancer in woodchucks and humans." T.M. Block, M.A. Comunale, M. Lowman, L.F. Steel, P.R. Romano, C. Fimmel, B.C. Tennant, W.T. London, A.A. Evans, B.S. Blumberg, R.A. Dwek, T.S. Mattu and A.S. Mehta (2005) Proc. Natl. Acad. Sci. USA, 102, 779-784.

The following examples illustrate the methodology for determining the glycosylation markers of disease and the use of databases in the detailed glycan analysis for hepatocellular carcinoma and for rheumatoid arthritis. However, it should be understood that the present invention is not limited thereto.

The invention is further illustrated by, though in no way limited to, the following examples.

### Example 1. Hepatocellular carcinoma in hepatitis C virus infected patients.

Glycosylation profiles of glycans released from whole serum of controls and hepatitis C virus (HCV) infected patients with hepatocellular carcinoma were compared to detect a potential glycosylation marker differentiating the two groups. Healthy control serum samples from two individuals, and one pooled sample were analyzed. A specific database containing NP-HPLC serum glycan profiles for both sialylated and neutral glycans was constructed and identified 38 glycans (Figure 4, Table 2). The same procedure was applied to patient sera and the glycosylation marker of hepatocellular carcinoma in HCV patients was identified by comparison the database of glycans released from whole serum of HCV infected patients with the database of glycans released from whole serum of healthy controls.

**Table 2. Glycan structures identified in serum of healthy control. Peak numbers correspond to peak numbers in figure 4. Abbreviations used: M5-9: GlcNAc₂Manₓ, where x is the number of mannoses; Ax: number of antenna, i.e. A2 is biantennary; Gx: number of galactose, [3] and [6] indicate which arm(3 or 6 linked) the galactose is attached to; Sx: the number of sialic acids; B: a bisecting GlcNAc; Fc: α1-6 core fucose. For example, peak 5 with a GU of 6.17 is Man5, this can also be written as GlcNAc₂Man₅; peak 33 is Man9 can be also written as GlcNAc₂Man₉.**

| **Peak Number** | **Structure** | **GU** |
|---|---|---|
| **1** | A2 | 5.47 |
| **2** | A2B | 5.76 |
| **3** | FcA2 | 5.90 |
| **4** | FcA2B | 6.15 |
| **5** | M5 | 6.17 |
| **6** | A2[6]G | 6.29 |
| **7** | A2B[6]G1 | 6.46 |
| **8** | A2[3]G1 | 6.48 |
| **9** | FcA2[6]G1 | 6.62 |
| **10** | A2B[3]G1 | 6.63 |
| **11** | FcA2[3]G1 | 6.78 |
| **12** | FcA2B[6]G1 | 6.83 |
| **13** | FcA2B[3]G1 | 6.90 |
| **14** | M6 | 7.06 |
| **15** | A2G2 | 7.13 |
| **16** | A2G1S1 | 7.14 |
| **17** | A2BG2 | 7.25 |
| **18** | A2BG1S1 | 7.47 |
| **19** | FcA2G2 | 7.50 |
| **20** | FcA2BG2 | 7.60 |
| **21** | FcA2BG1S1 | 7.71 |
| **22** | FcA2G1S1 | 7.75 |
| **23** | M7 | 7.93 |
| **24** | A2G2S1 | 8.02 |
| **25** | A2BG2S1 | 8.24 |
| **26** | FcA2G2S1 | 8.42 |
| **27** | FcA2BG2S1 | 8.61 |
| **28** | M8 | 8.77 |
| **29** | A2G2S2 | 882 |
| **30** | A2BG2S2 | 8.90 |
| **31** | FcA2G2S2 | 9.18 |
| **32** | FcA2BG2S2 | 9.25 |
| **33** | M9 | 9.50 |
| **34** | A3G3S2 | 9.78 |
| **35** | A3G3S3 | 10.11 |
| **36** | A3BG3S3 | 10.43 |
| **37** | FcA3G3S3 | 10.60 |
| **38** | FcA3BG3S3 | 10.65 |

Samples of serum from HCV infected patients with hepatocellular carcinoma were obtained from HCV infected patients with moderate or severe fibrosis/cirrhosis. Samples of healthy control serum were obtained as discarded clinical material from individuals undergoing routine health screening

Glycoproteins in reduced and denatured serum samples were attached to a hydrophobic PVDF membrane in a 96 well plate (Multiscreen_IP, 0.45µm hydrophobic, high polyvinyldene fluoride (PVDF) membranes, Millipore, Bedford, MA, USA) by simple filtration. The samples were then washed to remove contaminates, incubated with PNGaseF to release the glycans based on the methods described in Papac, D. I., et. al. Glycobiology 8: 445-54, 1998, and in Callewaert, N., et. al. Electrophoresis 25: 3128-31, 2004. The *N*-glycans were then washed from the bound protein, collected and dried down ready for fluorescent labeling.

Released glycans were labeled with 2-aminobenzamide (2-AB) fluorescent label with or without a commercial kit (from e.g. Ludger Ltd, Oxford, UK) as described in Bigge, J. C., Patel, T. P., Bruce, J. A., Goulding, P. N., Charles, S. M., and Parekh, R. B. (1995). Nonselective and efficient fluorescent labeling ofglycans using 2-amino benzamide and anthranilic acid. Analytical Biochemistry 230: 229-238*,* and run by normal phase high performance liquid chromatography (NP-HPLC) on a 4.6 x 250 mm TSK Amide-80 column (Anachem, Luton, UK) using a Waters 2695 separations module equipped with a Waters 2475 fluorescence detector (Waters, Milford, MA, USA) as described in Guile, G. R, Rudd, P. M., Wing, D. R., Prime, S. B., and Dwek, R. A. (1996). A rapid high-resolution high-performance liquid chromatographic method for separating glycan mixtures and analyzing oligosaccharide profiles. Analytical Biochemistry 240: 210-226. Prior to NP-HPLC analysis, glycans were sequentially digested with a series of exoglycosidases.

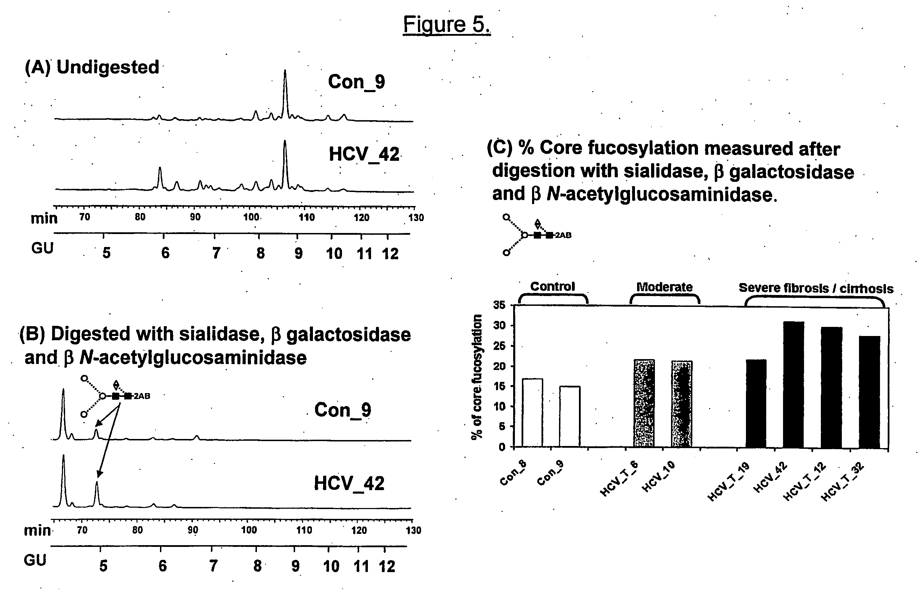

Figure 5 presents NP-HPLC profiles of glycans released from control sample Con_9 and from sample of HCV infected patient with hepatocellular carcinoma HCV_42. On figure 5, panel (A) corresponds to glycoprofiles of whole serum glycans not exposed to any exoglycosidase digestion, panel (B) to glycoprofiles following digestion with an array of α2-3,6,8-sialidase, β1-4 galactosidase and β-*N-*acetylglucosaminidase. Panel (C) of figure 5 demonstrates that the marker correlated with the diagnosis of hepatocellular carcinoma in HCV patients is the percentage of core fucosylated glycans measured after digestion with α2-3,6,8-sialidase, β1-4 galactosidase and β-*N*-acetylglucosaminidase. Healthy control sera contains between 15 and 17% of these glycans, while sera of HCV infected patients with hepatocellular carcinoma contained more than 19% of these glycans. The correlation was also observed between the stage of the disease and the percentage of core fucosylated glycans measured after digestion with α2-3,6,8-sialidase, β1-4 galactosidase and β-*N-*acetylglucosaminidase. HCV infected patients in moderate stage of hepatocellular carcinoma had the percentage of core fucosylated glycans measured after digestion with α2-3,6,8-sialidase, β1-4 galactosidase and β-*N*-acetylglucosaminidase of 20-22%, while patients in severe stages of disease, such as severe fibrosis/ cirrhosis, had this glycan marker above 25 % on average.

Conclusion: a glycosylation marker of hepatocellular carcinoma in HCV patients was identified by comparing glycosylation profiles of glycans released from whole serum of HCV patients with hepatocellular carcinoma and of glycans released from whole serum of healthy controls. The glycosylation marker of hepatocellular carcinoma in HCV patients is the percentage of core fucosylated glycans measured after digestion with α2-3,6,8-sialidase, β1-4 galactosidase and β-*N-*acetylglucosaminidase. The marker correlates with the disease diagnosis and the disease severity. Digestion of glycans with exoglycosidases amplifies/segregates the glycosylation marker of hepatocellular carcinoma in HCV patients.

### Example 2. Rheumatoid Arthritis

The measurement of the G0/triple-G1 ratio directly from undigested glycans released from whole serum was compared with the 'classic' measurement of the amount of G0 glycans as a percentage of the total glycans released from purified IgG after sialidase and fucosidase digestion. It has been shown that G0 released from purified IgG is disease(RA) specific marker that correlates with disease progression and that can be used as a prognostic indicator of the disease, see e.g. US patent No. 4,659,659 "*Diagnostic Method for Diseases Having an Arthritic Component*" to Dwek *et. al*. issued on April 21, 1987; Parekh et al., see "Association of Rheumatoid Arthritis and Primary Osteoarthritis with Changes in the Glycosylation Pattern of Total Serum IgG, "Nature, 316, pp. 452-457, 1985; and Parekh et. al. "Galactosylation of IgG Associated Oligosaccharides Is Reduced in Patients with Adult and Juvenile Onset Rheumatoid Arthritis and Is Related to Disease Activity," Lancet, No. 8592, vol. 1, pp. 966-969, 1988. This study is used to demonstrate that a direct measurement of glycans released from whole serum can be used as marker for rheumatoid arthritis without IgG purification by correlating G0/triple-G1 ratio from undigested glycans released from whole serum with the amount of G0 glycans as a percentage of the total glycans released from purified IgG.

### Selection of patient sample.

Control patient serum was pooled discarded clinical material from individuals undergoing routine employee health screening. RA patients were selected based on a combination of physician global activity score, rheumatoid factor seropositivity and active joint count

### IgG purification:

IgG was isolated from whole serum via affinity chromatography employing protein-G sepharose as described in "Antibodies: A laboratory manual, " Cold Spring Harbor Laboratory, Cold Spring Harbor, 1988, and P.L. Ey et. al. "Isolation of pure IgG1, IgG2a and IgG2b immunoglobulins from mouse serum using protein A-Sepharose," Molecular Immunology, vol. 15, pp. 429, 1978. Briefly, 100µl of whole serum was diluted with 300µl of 100mM Tris pH 8.0 and allowed to pass over a 1ml column of protein-G sepharose beads (Amersham Biosciences). Bound material was washed with 15 column volumes of 100mM Tris pH 8.0. IgG was eluted using 100mM glycine pH 2.6 buffer directly into 1/10 volume 1M Tris pH 8.0 and collected in 1ml fractions. Protein content of eluted fractions was determined by 280nM (UV) absorbance (Beckman Coulter Model DU640 spectrophotometer). Protein containing eluted fractions were pooled and dialyzed into phosphate buffered saline. IgG presence in eluted fractions was confirmed via 10% polyacryl amide gel electrophoresis (PAGE) under reducing conditions (as described, e.g., in Laemmli, "Cleavage of structural proteins during the assembly of the head of bacteriophage T4," Nature, : 227, 680-685, 1970 and via western blot (Current Protocols in Immunology. John Wiley and Sons, 1994) utilizing horseradish-peroxidase conjugated donkey-antihuman IgG (Jackson Immunochemicals) and visualized with Western Lightning Chemiluminescence Reagent Plus (Perkin Elmer). Quantitative depletion of serum IgG in column flow through material was confirmed via western blot analysis.

### Glycans release:

Glycans were released from purified IgG by running the reduced and alkylated sample on sodium-dodecyl sulphate polyacryl amide gel electrophoresis (SDS-PAGE), cutting out the heavy chain and digesting with peptide N-glycosidase F (PNOaseF) as described in Küster, B., Wheeler, S. F., Hunter, A. P., Dwek, R. A., and Harvey, D. J. (1997). Sequencing of N-linked oligosaccharides directly from protein gels: in-gel deglycosylation followed by matrix-assisted laser desorption/ionization mass spectrometry and normal-phase high-performance liquid chromatography. Analytical Biochemistry 250: 82-101. Glycans were released with PNGaseF from 5 µl of whole sera after binding the reduced and alkylated serum to MultiScreen_IP, 0.45µm hydrophobic, high protein binding polyvinylidene fluoride (PVDF) membranes in a 96 well plate format (Millipore, Bedford, MA, USA). Released glycans were labeled with 2AB fluorescent label (Ludger Ltd, Oxford, UK) as described in Bigge, J. C., Patel, T. P., Bruce, J. A., Goulding, P. N., Charles, S. M., and Parekh, R. B. (1995). Nonselective and efficient fluorescent labeling of glycans using 2-amino benzamide and anthranilic acid. Analytical Biochemistry 230: 229-238*,* and run by normal phase high performance liquid chromatography (NP-HPLC) on a 4.6 x 250 mm TSK Amide-80 column (Anachem, Luton, UK) using a Waters 2695 separations module equipped with a Waters 2475 fluorescence detector (Waters, Milford, MA, USA) as described in Guile, G. R, Rudd, P. M., Wing, D. R, Prime, S. B., and Dwek, R. A. (1996). A rapid high-resolution high-performance liquid chromatographic method for separating glycan mixtures and analyzing oligosaccharide profiles. Analytical Biochemistry 240: 210-226. Purified, 2AB labeled IgG heavy chain glycans were also digested with sialidase and fucosidase to reduce all the structures to G0, G1 or G2 +/- bisect, then run on NP-HPLC. [G0 denotes no galactose; G1, one galactose; and, G2 two galactose, all on biantennary complex N-glycans.]

### Statistical analysis.

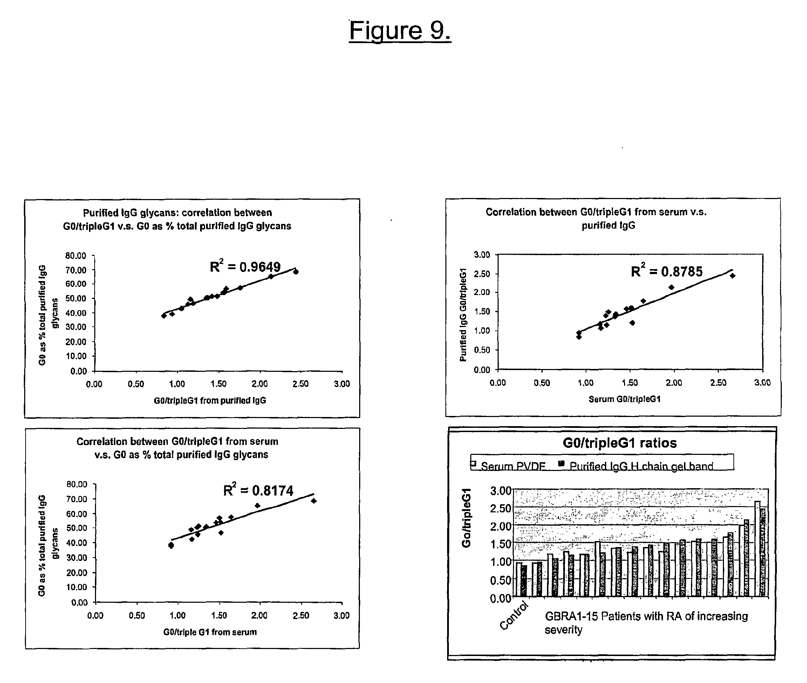

All the data for glycan ratios are listed in Table 3. Left top, left bottom, right top panels of figure 9 are plots showing correlations between these data. The R² values were obtained by linear regression analysis using Microsoft Excel.

### Experimental results.

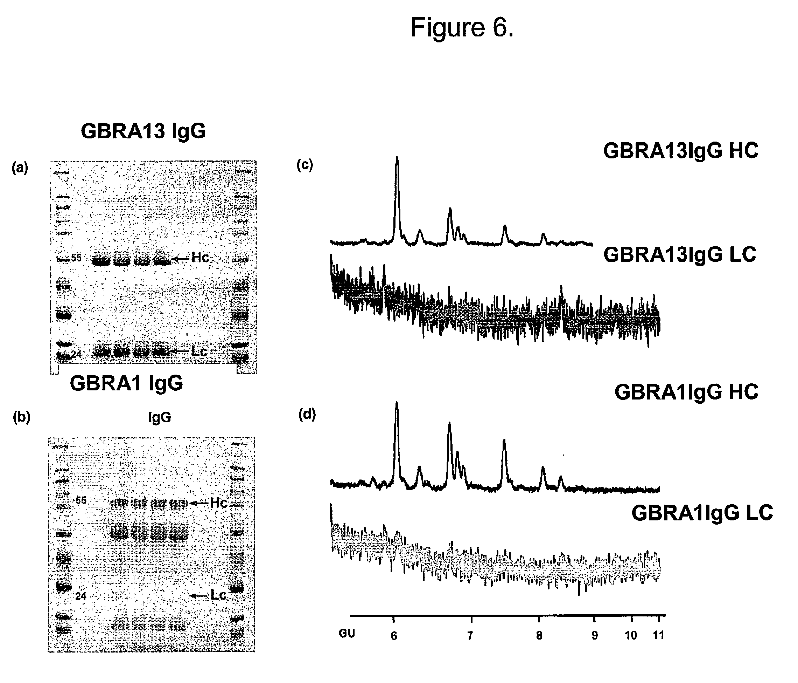

Figure 6 shows SDS-PAGE and NP-HPLC profiles from samples GBRA13 and GBRA1. In particular, insets (a) and (b) of figure 6 provide SDS-PAGE gel pictures of the purified IgGs from the respective samples separated into heavy (H) and light (L) chain bands. Insets (c) and (d) of figure 6 provide NP-HPLC profiles for heavy and light chain glycans released from the gel bands shown in (a) and (b) and not subjected to digestion with sialidase and fucosidase. Since no glycans were detected on the light chain, only the heavy chain was required for analysis.

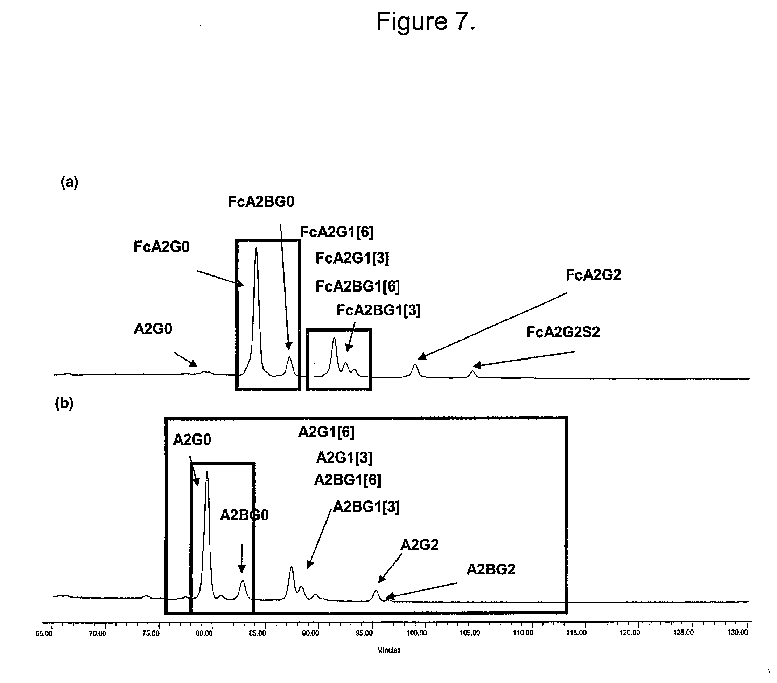

Figure 7 illustrates the details of (a) the measurement of the G0/triple-G1 ratio directly from undigested glycans released from purified IgG and (b) the 'classic' measurement of the ratio G0 glycans to the total glycans released from purified IgG and digested with sialidase and fucosidase. In particular, Figure 7 shows NP-HPLC profiles from the sample GBRA15. Each peak corresponds to certain glycan(s). The peaks in each profile are integrated to give the area under the curve for each peak. In the measurement of the G0/triple-G1 ratio, the area under the peaks corresponding to the G0 glycans (left box of the inset (a) of figure 7) are divided by the area under the triplet of peaks corresponding to the G1 glycans (right box of the inset (a) of figure 7). As the vast majority of glycans found in these experiments were core fucosylated, only core fucosylated glycans were included in these measurements, i.e. the ratio G0/triple-G1 is actually the peak area of FcA2G0 divided by the peak area of FcA2G1[6]+FcA2G1[3]+FcA2BG1[6]+FcA2BG1[3] (which elutes as a triplet).

In the 'classic' measurement, the area under the peaks corresponding to the G0 peaks is divided by the total area under all the peaks in the profile and expressed as a percentage.

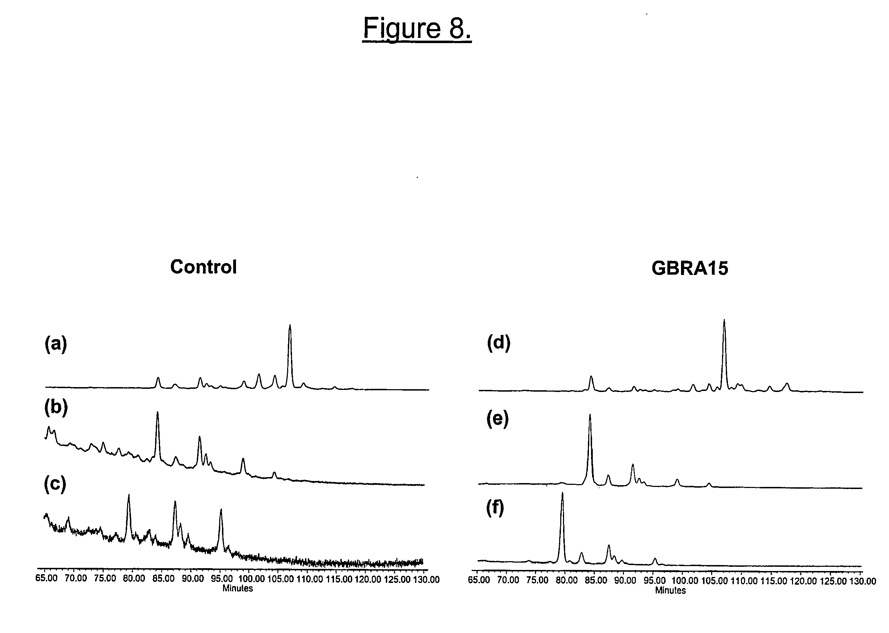

Figure 8 illustrates NP-HPLC profiles of control sample and the sample GBRA15. Particularly, insets (a) and (d) show glycans released from whole sera of the respective samples, insets (b) and (e) show undigested heavy chain glycans released from respective purified IgGs, insets (c) and (f) show heavy chain glycans released from respective purified IgGs and digested with sialidase and fucosidase.

Table 3 lists the ratios of the G0 to triple-G1 peak from whole serum and purified IgG from the same serum samples from 15 RA patients and one pooled control. The 'classic' measurement of the amount of G0 glycans as a percentage of the total glycans (G0+G1+G2) from purified IgG is also shown. Comparing the results of the two different measurements taken from purified IgG, a high correlation (R²=0.9649) is found, indicating that the ratio G0/triple-G1 is as a good measurement as the 'classic' measurement of the percentage of G0 glycans in total glycan pool (Figure 9, left top panel). Comparing the G0/triple-G1 ratio between purified IgG and whole serum glycans gives a correlation of R²=0.8785 (Figure 9, right top panel), whilst comparing the G0/triple-G1 ratio from whole serum glycans with the percentage G0 glycans from purified IgG gives a correlation of R²=0.8174 (Figure 9, left bottom panel). Figure 9, right bottom panel, is a histogram showing the GO/triple-G1 ratios for all serum and IgG samples.

**Table 3**

| **Patient i.d.** | **undigested G0 / triple-G1** | | **G0 as % of TOTAL digested IgG glycans** |
|---|---|---|---|
| | **Glycans released from Serum using PVDF** | **Glycans released from purified IgG using SDS-PAGE** | |
| Control | 0.92 | 0.84 | 37.40 |
| GBRA1 | 0.92 | 0.94 | 38.43 |
| GBRA2 | 1.17 | 1.05 | 42.26 |
| GBRA3 | 1.24 | 1.13 | 45.43 |
| GBRA4 | 1.16 | 1.16 | 48.74 |
| GBRA5 | 1.53 | 1.19 | 46.26 |
| GBRA6 | 1.33 | 1.35 | 49.94 |
| GBRA7 | 1.23 | 1.37 | 50.18 |
| GBRA8 | 1.34 | 1.42 | 50.74 |
| GBRA9 | 1.25 | 1.48 | 51.14 |
| GBRA10 | 1.46 | 1.56 | 53.50 |
| GBRA11 | 1.52 | 1.58 | 54.13 |
| GBRA12 | 1.51 | 1.59 | 56.59 |
| GBRA13 | 1.65 | 1.76 | 56.98 |
| GBRA14 | 1.97 | 2.13 | 65.16 |
| GBRA15 | 2.66 | 2.44 | 68.28 |

### Conclusion.

The use of the high throughput PVDF membrane 96 well plate format with only 5µl of whole serum being used to obtain glycans for a direct measurement of the G0/triple-G1 ratio has been demonstrated in place of the more lengthy procedure of measuring the percentage of G0 glycans in the glycans released from purified IgG determined after exoglycosidase treatment, as an indicator of RA disease state. Thus, to monitor the RA disease state, one can efficiently reduce working hours from sample preparation to results by using the PVDF membrane method with whole serum as well as reducing the amount of material (serum) used.

Although the foregoing refers to particular preferred embodiments, it will be understood that the present invention is not so limited. It will occur to those of ordinary skill in the art that various modifications may be made to the disclosed embodiments and that such modifications are intended to be within the scope of the present invention.

## Claims

1. An *in vitro* method of determining one or more glycosylation markers of a disease, comprising the steps of:
- providing a diseased sample and a control sample, wherein the diseased sample is a sample from a subject diagnosed with the disease and the control sample is a sample from a healthy control;
- immobilising total glycoproteins from the diseased sample and from the control sample on a protein binding membrane or in a gel without separating the gel into bands;
- releasing a glycan pool of the immobilised total glycoproteins of the diseased sample and releasing a glycan pool of the immobilised total glycoproteins of the control sample from the gel or the protein binding membrane without having purified a specific glycoprotein and without exposing the diseased sample and the control sample to hydrazinolysis;
- measuring a glycosylation profile of the glycan pool from the diseased sample and measuring a glycosylation profile of the glycan pool from the control sample using chromatography, mass spectrometry or a combination thereof; and
- comparing the glycosylation profile of the diseased sample with the glycosylation profile of the control sample to determine one or more glycosylation markers of the disease.

2. A method as claimed in claim 1 wherein the sample is a sample of a body fluid or a body tissue.

3. A method as claimed in claim 2 wherein the body fluid is whole serum.

4. A method as claimed in any one of claims 1 to 3 wherein the glycans are *N*-linked glycans.

5. A method as claimed in any one of claims 1 to 3 wherein the glycans are *O*-linked glycans.

6. A method as claimed in any one of claims 1 to 5 wherein the method includes a further step of labeling the glycans of the glycan pool with a radioactive or a fluorescent label prior to measuring the glycosylation profile.

7. A method as claimed in any one of claims 1 to 6 further comprising selecting a best glycosylation marker out of the glycosylation markers, wherein the best glycosylation marker has a highest correlation with one or more parameters of the disease.

8. A method as claimed in any one of claims 1 to 7 further comprising a step of segregating and/or amplifying the one or more glycosylation markers by digesting the glycans with one or more exoglycosidases.

9. A method as claimed in claim 8 wherein said digesting is performed sequentially.

10. A method as claimed in claim 8 wherein said digesting is digesting with an array comprising the one or more exoglycosidases.

11. A method as claimed in any one of claims 1 to 10 further comprising using a database to make preliminary and final assignments of structures of the glycans.

12. A method as claimed in any one of claims 1 to 11 further comprising using the one or more glycosylation markers of the disease to diagnose, prognose and/or monitor the disease.

13. An *in vitro* method for diagnosing and monitoring a disease in a subject comprising the steps of:
- providing a sample of body fluid or a body tissue of the subject;
- immobilising total glycoproteins from the sample on a protein binding membrane or in a gel without separating the gel into bands;
- releasing a glycan pool of the immobilised total glycoproteins of the sample from the gel or the protein binding membrane without having purified a specific glycoprotein and without exposing the sample to hydrazinolysis;
- measuring a glycosylation profile of the glycan pool; and
- determining a clinical status of the subject from one or more glycosylation markers of the disease in the glycosylation profile.

14. A method as claimed in claim 13 wherein the disease is cancer, an autoimmune disease or a congenital disorder of glycosylation.

15. A method as claimed in claim 14 wherein the autoimmune disease is rheumatoid arthritis.

16. A method as claimed in any one of claims 13 to 15 wherein the body fluid is whole serum.

17. A method as claimed in claim 16 when dependent on claim 15 wherein the glycosylation marker is a ratio between G0 glycans and triple-G1 glycans.

18. A method as claimed in any one of claims 13 to 16 further comprising a step of segregating and/or amplifying the one or more glycosylation markers by digesting the glycans with one or more exoglycosidases.

## Patentansprüche

1. Ein In-vitro-Verfahren zum Bestimmen von einem oder mehreren Glykosylierungsmarkern einer Erkrankung, das die folgenden Schritte beinhaltet:
- Bereitstellen einer befallenen Probe und einer Kontrollprobe, wobei die befallene Probe eine Probe von einem mit der Erkrankung diagnostizierten Versuchsobjekt ist und die Kontrollprobe eine Probe von einem gesunden Versuchsobjekt ist;
- Immobilisieren von Gesamtglykoproteinen aus der befallenen Probe und aus der Kontrollprobe auf einer proteinbindenden Membran oder in einem Gel, ohne das Gel in Bande zu trennen;
- Freisetzen eines Glykanpools der immobilisierten Gesamtglykoproteine der befallenen Probe und Freisetzen eines Glykanpools der immobilisierten Gesamtglykoproteine der Kontrollprobe aus dem Gel oder der proteinbindenden Membran, ohne ein spezifisches Glykoprotein gereinigt zu haben und ohne die befallene Probe und die Kontrollprobe Hydrazinolyse auszusetzen;
- Messen eines Glykosylierungsprofils des Glykanpools aus der befallenen Probe und Messen eines Glykosylierungsprofils des Glykanpools aus der Kontrollprobe unter Verwendung von Chromatographie, Massenspektrometrie oder einer Kombination davon; und
- Vergleichen des Glykosylierungsprofils der befallenen Probe mit dem Glykosylierungsprofil der Kontrollprobe, um einen oder mehrere Glykosylierungsmarker der Erkrankung zu bestimmen.

2. Verfahren gemäß Anspruch 1, wobei die Probe eine Probe einer Körperflüssigkeit oder eines Körpergewebes ist.

3. Verfahren gemäß Anspruch 2, wobei die Körperflüssigkeit Vollserum ist.

4. Verfahren gemäß einem der Ansprüche 1 bis 3, wobei die Glykane *N*-verknüpfte Glykane sind.

5. Verfahren gemäß einem der Ansprüche 1 bis 3, wobei die Glykane *O*-verknüpfte Glykane sind.

6. Verfahren gemäß einem der Ansprüche 1 bis 5, wobei das Verfahren einen weiteren Schritt der Markierung der Glykane des Glykanpools mit einer radioaktiven oder einer fluoreszierenden Markierung vor der Messung des Glykosylierungsprofils umfasst.

7. Verfahren gemäß einem der Ansprüche 1 bis 6, das ferner das Auswählen eines besten Glykosylierungsmarkers aus den Glykosylierungsmarkern beinhaltet, wobei der beste Glykosylierungsmarker eine höchste Korrelation mit einem oder mehreren Parametern der Erkrankung aufweist.

8. Verfahren gemäß einem der Ansprüche 1 bis 7, das ferner einen Schritt des Segregierens und/oder Amplifizierens des einen oder der mehreren Glykosylierungsmarker durch das Digerieren der Glykane mit einer oder mehreren Exoglykosidasen beinhaltet.

9. Verfahren gemäß Anspruch 8, wobei das Digerieren sequentiell durchgeführt wird.

10. Verfahren gemäß Anspruch 8, wobei das Digerieren Digerieren mit einer Anordnung ist, die die eine oder mehrere Exoglykosidasen beinhaltet.

11. Verfahren gemäß einem der Ansprüche 1 bis 10, das ferner die Verwendung einer Datenbank beinhaltet, um vorläufige und endgültige Zuordnungen von Strukturen der Glykane vorzunehmen.

12. Verfahren gemäß einem der Ansprüche 1 bis 11, das ferner die Verwendung des einen oder mehrerer Glykosylierungsmarker der Erkrankung beinhaltet, um die Erkrankung zu diagnostizieren, prognostizieren und/oder zu überwachen.

13. Ein In-vitro-Verfahren zum Diagnostizieren und Überwachen einer Erkrankung bei einem Versuchsobjekt, das die folgenden Schritte beinhaltet:
- Bereitstellen einer Probe von Körperflüssigkeit oder Körpergewebe des Versuchsobjekts;
- Immobilisieren von Gesamtglykoproteinen aus der Probe auf einer proteinbindenden Membran oder in einem Gel, ohne das Gel in Bande zu trennen;
- Freisetzen eines Glykanpools der immobilisierten Gesamtglykoproteine der Probe aus dem Gel oder der proteinbindenden Membran, ohne ein spezifisches Glykoprotein gereinigt zu haben und ohne die Probe Hydrazinolyse auszusetzen;
- Messen eines Glykosylierungsprofils des Glykanpools; und
- Bestimmen eines klinischen Status des Versuchsobjekts aus einem oder mehreren Glykosylierungsmarkern der Erkrankung in dem Glykosylierungsprofil.

14. Verfahren gemäß Anspruch 13, wobei die Erkrankung Krebs, eine Autoimmunerkrankung oder eine kongenitale Störung der Glykosylierung ist.

15. Verfahren gemäß Anspruch 14, wobei die Autoimmunerkrankung rheumatoide Arthritis ist.

16. Verfahren gemäß einem der Ansprüche 13 bis 15, wobei die Körperflüssigkeit Vollserum ist.

17. Verfahren gemäß Anspruch 16, wenn abhängig von Anspruch 15, wobei der Glykosylierungsmarker ein Verhältnis zwischen GO-Glykanen und dreifach-G1-Glykanen ist.

18. Verfahren gemäß einem der Ansprüche 13 bis 16, das ferner einen Schritt des Segregierens und/oder Amplifizierens des einen oder der mehreren Glykosylierungsmarker durch das Digerieren der Glykane mit einer oder mehreren Exoglykosidasen beinhaltet.

## Revendications

1. Une méthode in vitro pour déterminer un ou plusieurs marqueurs de glycosylation d'une maladie, comprenant les étapes de :
- fournir un échantillon malade et un échantillon témoin, l'échantillon malade étant un échantillon prélevé chez un sujet diagnostiqué avec la maladie et l'échantillon témoin étant un échantillon provenant d'un groupe témoin en bonne santé ;
- immobiliser des glycoprotéines totales provenant de l'échantillon malade et de l'échantillon témoin sur une membrane de fixation de protéines ou dans un gel sans séparer le gel en bandes ;
- libérer une fraction groupée de glycanes des glycoprotéines totales immobilisées de l'échantillon malade et libérer une fraction groupée de glycanes des glycoprotéines totales immobilisées de l'échantillon témoin du gel ou de la membrane de fixation de protéines sans avoir purifié une glycoprotéine spécifique et sans exposer l'échantillon malade et l'échantillon témoin à une hydrazinolyse ;
- mesurer un profil de glycosylation de la fraction groupée de glycanes provenant de l'échantillon malade et mesurer un profil de glycosylation de la fraction groupée de glycanes provenant de l'échantillon témoin à l'aide d'une chromatographie, d'une spectrométrie de masse ou d'une combinaison de celles-ci ; et
- comparer le profil de glycosylation de l'échantillon malade avec le profil de glycosylation de l'échantillon témoin afin de déterminer un ou plusieurs marqueurs de glycosylation de la maladie.

2. Une méthode telle que revendiquée dans la revendication 1 dans laquelle l'échantillon est un échantillon d'un liquide organique ou d'un tissu organique.

3. Une méthode telle que revendiquée dans la revendication 2 dans laquelle le liquide organique est du sérum entier.

4. Une méthode telle que revendiquée dans n'importe laquelle des revendications 1 à 3 dans laquelle les glycanes sont des glycanes à liaison N.

5. Une méthode telle que revendiquée dans n'importe laquelle des revendications 1 à 3 dans laquelle les glycanes sont des glycanes à liaison *O*.

6. Une méthode telle que revendiquée dans n'importe laquelle des revendications 1 à 5 dans laquelle la méthode inclut une étape supplémentaire consistant à étiqueter les glycanes de la fraction groupée de glycanes avec une étiquette radioactive ou fluorescente avant de mesurer le profil de glycosylation.

7. Une méthode telle que revendiquée dans n'importe laquelle des revendications 1 à 6 comprenant de plus le fait de sélectionner un meilleur marqueur de glycosylation parmi les marqueurs de glycosylation, le meilleur marqueur de glycosylation ayant une corrélation la plus grande avec un ou plusieurs paramètres de la maladie.

8. Une méthode telle que revendiquée dans n'importe laquelle des revendications 1 à 7 comprenant de plus une étape consistant à ségréger et / ou à amplifier ces un ou plusieurs marqueurs de glycosylation par digestion des glycanes par une ou plusieurs exoglycosidases.

9. Une méthode telle que revendiquée dans la revendication 8 dans laquelle ladite digestion est effectuée de façon séquentielle.

10. Une méthode telle que revendiquée dans la revendication 8 dans laquelle ladite digestion est une digestion par une matrice comprenant ces une ou plusieurs exoglycosidases.

11. Une méthode telle que revendiquée dans n'importe laquelle des revendications 1 à 10 comprenant de plus le fait d'utiliser une base de données pour réaliser des attributions préliminaires et finales de structures des glycanes.

12. Une méthode telle que revendiquée dans n'importe laquelle des revendications 1 à 11 comprenant de plus le fait d'utiliser ces un ou plusieurs marqueurs de glycosylation de la maladie afin de diagnostiquer, de pronostiquer et / ou de surveiller la maladie.

13. Une méthode in vitro pour diagnostiquer et surveiller une maladie chez un sujet comprenant les étapes de :
- fournir un échantillon de liquide organique ou de tissu organique du sujet ;
- immobiliser des glycoprotéines totales provenant de l'échantillon sur une membrane de fixation de protéines ou dans un gel sans séparer le gel en bandes ;
- libérer une fraction groupée de glycanes des glycoprotéines totales immobilisées de l'échantillon du gel ou de la membrane de fixation de protéines sans avoir purifié une glycoprotéine spécifique et sans exposer l'échantillon à une hydrazinolyse ;
- mesurer un profil de glycosylation de la fraction groupée de glycanes ; et
- déterminer un statut clinique du sujet à partir d'un ou plusieurs marqueurs de glycosylation de la maladie dans le profil de glycosylation.

14. Une méthode telle que revendiquée dans la revendication 13 dans laquelle la maladie est un cancer, une maladie auto-immune ou un trouble congénital de glycosylation.

15. Une méthode telle que revendiquée dans la revendication 14 dans laquelle la maladie auto-immune est de l'arthrite rhumatoïde.

16. Une méthode telle que revendiquée dans n'importe laquelle des revendications 13 à 15, dans laquelle le liquide organique est du sérum entier.

17. Une méthode telle que revendiquée dans la revendication 16 lorsqu'elle dépend de la revendication 15 dans laquelle le marqueur de glycosylation est un rapport entre des glycanes G0 et des glycanes triple-G1.

18. Une méthode telle que revendiquée dans n'importe laquelle des revendications 13 à 16 comprenant de plus une étape consistant à ségréger et / ou à amplifier ces uns ou plusieurs marqueurs de glycosylation par digestion des glycanes par une ou plusieurs exoglycosidases.
